(19) Europäisches Patentamt
European Patent Office
Office européen des brevets

(11) EP 2 355 775 B1

(12) **EUROPEAN PATENT SPECIFICATION**

(45) Date of publication and mention of the grant of the patent:
**21.06.2017 Bulletin 2017/25**

(21) Application number: **09775447.7**

(22) Date of filing: **08.12.2009**

(51) Int Cl.:
***A61K 8/02*** (2006.01)
***A61K 8/46*** (2006.01)
***A61K 8/44*** (2006.01)
***A61Q 5/12*** (2006.01)
***A61K 8/04*** (2006.01)
***A61K 8/898*** (2006.01)
***A61K 8/34*** (2006.01)
***A61K 8/81*** (2006.01)
***A61Q 5/02*** (2006.01)
***A61K 8/73*** (2006.01)
***A61K 8/891*** (2006.01)

(86) International application number:
**PCT/US2009/067133**

(87) International publication number:
**WO 2010/077653 (08.07.2010 Gazette 2010/27)**

(54) **PERSONAL CARE COMPOSITION IN THE FORM OF AN ARTICLE HAVING A HYDROPHOBIC SURFACE-RESIDENT COATING**

KÖRPERPFLEGEZUSAMMENSETZUNG IN FORM EINES ARTIKELS MIT WASSERABWEISENDER OBERFLÄCHENBESCHICHTUNG

COMPOSITION DE SOIN D’HYGIÈNE PERSONNELLE SOUS LA FORME D’UN ARTICLE POSSÉDANT UN REVÊTEMENT HYDROPHOBE EN SURFACE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **08.12.2008 US 120790 P**

(43) Date of publication of application:
**17.08.2011 Bulletin 2011/33**

(73) Proprietor: **The Procter & Gamble Company**
**Cincinnati, OH 45202 (US)**

(72) Inventors:
- **GLENN, Robert, Wayne, Jr.**
**Liberty Township, Ohio 45044 (US)**
- **HEINRICH, James, Merle**
**Fairfield, Ohio 45014 (US)**
- **KAUFMAN, Kathy, Mary**
**Cincinnati, Ohio 45247 (US)**
- **HUTCHINS, Virginia, Tzung-Hwei**
**Cincinnati, Ohio 45220 (US)**
- **DUBOIS, Zerlina, Guzdar**
**Mason, Ohio 45040 (US)**
- **LI, Jianjun, Justin**
**West Chester, Ohio 45069 (US)**
- **LABITZKE, Kevin, M.**
**Hamilton, Ohio 45011 (US)**
- **TROKHAN, Darren, Paul**
**Hamiltion, Ohio 45013 (US)**
- **DUFRESNE, Tom, Edward**
**Morrow, Ohio 45152 (US)**
- **SCHECHTMAN, Lee, Arnold**
**Fairfield, Ohio 45014 (US)**

(74) Representative: **Briatore, Andrea et al**
**Procter & Gamble Service GmbH**
**IP Department**
**Frankfurter Strasse 145**
**61476 Kronberg im Taunus (DE)**

(56) References cited:
**WO-A1-2005/075547**
**WO-A2-03/037294**
**US-A- 6 106 849**
**US-A1- 2008 152 894**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 355 775 B1

## Description

FIELD OF THE INVENTION

The present invention relates to personal care compositions, especially those personal care compositions in the form of an article that is a porous, dissolvable solid structure. The article has a hydrophobic surface-resident coating that can provide enhanced deposition efficiency of hydrophobic actives contained therein.

BACKGROUND OF THE INVENTION

The majority of personal care products in the market today are sold as liquid products. While widely used, liquid products have disadvantages in terms of packaging, storage, transportation, and convenience of use. Such products commonly contain hydrophobic benefit agents intended for surface deposition on the hair and/or skin (e.g., hair conditioning agents such as silicone). Because such products typically comprise a substantial amount of water in the formula, the incorporation of such hydrophobic benefit agents can be difficult, requiring formulation techniques such as emulsification in order to deliver the hydrophobic active. Furthermore, because such products are used in aqueous environments (e.g., shower), the pre-emulsified hydrophobic benefit agents have a tendency to rinse off the hair and/or skin before they can be sufficiently deposited, leading to less than optimal deposition efficiency and thus wasted benefit agents.

Thus, there is thus a need for a personal care product that can deliver enhanced levels of hydrophobic active surface deposition to skin and/or hair. There is also a need for such a personal care product to be provided in a non-aqueous form.

SUMMARY OF THE INVENTION

The present invention meets the aforementioned needs. The present invention provides a dissolvable solid personal care product that can be conveniently and quickly dissolved in the palm of the consumer to reconstitute a liquid product for ease of application to hair/skin while providing sufficient topical delivery of hydrophobic benefit agents for whole head hair and/or whole body skin applications.

In one aspect, the invention provides a personal care article in the form of a porous dissolvable solid structure, comprising:

(a) from about 0% to about 10% ionic surfactant;
(b) from about 1% to about 60% of a hydrophobic surface-resident coating, wherein said coating comprises a non-surfactant cosmetic benefit agent;
(c) from about 15% to about 70% polymeric structurant, wherein said polymeric structurant has a weighted average molecular weight of from about 40,000 to about 500,000; and
(d) from about 1% to about 30% plasticizer

wherein said article has a density of from about 0.05 g/$cm^3$ to about 0.4 g/$cm^3$.

In a particular embodiment, the article is a lathering personal care article in the form of a porous dissolvable solid structure, comprising:

(a) from about 23% to about 75% ionic surfactant;
(b) from about 1% to about 60% of a hydrophobic surface-resident coating, wherein said coating comprises a non-surfactant cosmetic benefit agent;
(c) from about 10% to about 50% water soluble polymer; and
(d) from about 1% to about 30% plasticizer;

wherein said article has a density of from about 0.05 g/cm3 to about 0.4 g/cm3.

It has been surprisingly found that improved deposition efficiency of actives (e.g., silicone) can be achieved by coating or including the active as a coating on the article (e.g., adsorbed onto the solid/air interfaces of the structure of the article) instead of adding the active as a conventional emulsion during article making, i.e., within the continuous solid structure. This is surprising as silicones normally need to be dispersed/emulsified into a liquid (e.g., especially a high viscosity silicone "gum" as demonstrated in the examples having a viscosity of 330,000 centistokes) to generate deposition onto hair, especially without producing combing negatives.

While not being bound by theory, it is believed that the predominantly open-celled porous articles provide a continuous and accessible high surface area "scaffold" (a 3-D network of "struts") for the oil to spread across, creating a high surface area microscopically thin coating. It is believed that the resulting high oil:solid surface area enables the

in-situ dispersion of the oil upon dissolution of the porous solid with water for uniform deposition and tactile performance as is demonstrated herein. Additionally, it is believed that this coating approach minimizes the opportunity for oil:surfactant intimacy (less emulsification) which facilitates greater deposition efficiency. Moreover, these benefits can be achieved without phase instability challenges over the shelf-life of the product.

Furthermore, the present invention provides a way to deliver any water-insoluble fluid, liquid, and/or gum without emulsification (e.g., alternative silicones, triglycerides, hydrocarbon oils, perfume oils, etc.). This is especially useful in the case of the volatile actives, such as perfumes, some of which would be lost to the atmosphere during the drying process utilized during the preparation of the porous dissolvable solid structures as described herein..

In another aspect, the present invention provides a method for making the article, comprising: (1) Preparing a processing mixture comprising surfactant, dissolved polymer structurant, plasticizer and optionally other optional ingredients; (2) Aerating the processing mixture by introducing a gas into the mixture to form an aerated wet mixture; (3) Forming the aerated wet mixture into a desired one or more shapes; (4) Drying the aerated wet mixture to a desired final moisture content (e.g., from about 0.1 to 25% moisture, by addition of energy) to form the article in the form of a porous dissolvable solid structure; and (5) Applying a hydrophobic surface-resident coating to the article such that said surface-resident coating is adsorbed to the solid/air interface of said article to form a coated article. The coating can comprise a non-surfactant cosmetic benefit agent (e.g., a cosmetic active).

In addition, the present invention provides a means by which the article can be customized at the point of sale or usage, either by the end consumer or by a professional, such as in a salon. This can provide for a customized experience, allowing the consumer to choose among various benefit agents to achieve one or more desired cosmetic effects (e.g., choice of fragrance, hair conditioning agents). In this embodiment, the method of making the article comprises: (1) obtaining a personal care article in the form of a porous dissolvable solid structure described herein, and (2) applying a hydrophobic surface-resident coating comprising a non-surfactant benefit agent that is adsorbed onto the solid/air interface of the said article.

Any suitable application method can be used to apply the hydrophobic benefit agent to the article such that it forms a surface-resident coating that is absorbed to the article's surface. For instance, it can be sprayed, spread, dropped, printed, sandwiched between different articles or different portions of the same article, layered, injected, rolled on, or dipped. The hydrophobic benefit agent can be applied over portions or entire regions of the article's exterior surface, and can be applied in a manner to adorn, decorate, form a logo, design, etc.

The hydrophobic surface-resident coating is "flowable," meaning that it is in a liquid state at the time of application to the article, and has a viscosity of less than 3,000,000 centistokes at temperatures from 25°C to 80°C.

The coating is also hydrophobic, having a Vaughan's solubility parameter ("VSP") of from 0 $(cal/cm^3)^{1/2}$ to 10 $(cal/cm^3)^{1/2}$, in particular embodiments from 1 $(cal/cm^3)^{1/2}$ to 10 $(cal/cm^3)^{1/2}$, in some embodiments from about 3 to about 10 $(cal/cm^3)^{1/2}$, in other embodiments from about 5 to about 10 $(cal/cm^3)^{1/2}$, and comprises a non-surfactant cosmetic benefit agent. The hydrophobic coating can be in any suitable form, such as a liquid, a complex liquid, an oil, an oil with a dispersed phase (e.g., either solid or liquid), an emulsion, or mixtures thereof. Vaughan's solubility parameter is determined as defined by Vaughan in Cosmetics and Toiletries, Vol. 103. Non-limiting examples of hydrophobic moisturizing materials having VSP values ranging from about 5 to about 10 include the following: Dimethicone 5.92, Squalene 6.03, Petrolatum 7.33, Isopropyl Palmitate 7.78, Isopropyl Myristate 8.02, Castor Oil 8.90, Cholesterol 9.55, Safflower Oil 6.42, and White Mineral Oil 7.09, as reported in Solubility, Effects in Product, Package, Penetration and Preservation, C. D. Vaughan, Cosmetics and Toiletries, Vol. 103, October 1988.

In particular embodiments, the personal care article has a basis weight of from about 125 grams/$m^2$ to about 3,000 grams/$m^2$, in one embodiment from about 200 grams/$m^2$ to about 2,500 grams/$m^2$, in another embodiment from about 300 grams/$m^2$ to about 2,000 grams/$m^2$, and in yet another embodiment from about 400 grams/$m^2$ to about 1,500 grams/$m^2$.

In specific embodiments, the personal care article has a thickness of from about 0.5 mm to about 10 mm, in a particular embodiment from about 1 mm to about 9 mm, in another embodiment from about 2 mm to about 8 mm, and in a further embodiment from about 3 mm to about 7 mm.

In one embodiment, at least one of the one or more water-soluble polymers is chosen such that a 2% by weight solution of the water-soluble polymer gives a viscosity at 20°C of from about 4 centipoise to about 80 centipoise.

In one embodiment, the personal care article has very low lather volumes, which can be from about 0 ml to about 20 ml, in other embodiments from about 0 ml to about 15 ml, and in still other embodiments from about 0 ml to about 10 ml. In alternate embodiments, the personal care article is a lathering article, having a lather volume greater than 20 ml.

These and other aspects and advantages of the present invention will become evident to those skilled in the art from a reading of the following detailed description of the invention.

BRIEF DESCRIPTION OF THE DRAWINGS

While the specification concludes with claims which particularly point out and distinctly claim the present invention, it is believed that the present invention will be better understood from the following description of embodiments, taken in conjunction with the accompanying drawings and wherein:

FIGs. 1 - 4 are representative cross-sectional comparisons of the original SEM image (on the left) with the elemental map of silicone of the same cross-sectional view (on the right) from Example 12 herein. The presence of silicon is shown as white. From these figures one can clearly see that the silicon (representative of silicone) resides predominantly at the porous solid/air interfaces versus being distributed throughout the interior of the freshly cut solid cell walls. These figures demonstrate the presence of the silicone cosmetic active as a surface resident coating across several solid/air interfaces (both at the upper surface and within exposed cavities within the solid).

DETAILED DESCRIPTION OF THE INVENTION

Definitions

In all embodiments of the present invention, all concentrations and percentages are by weight of the total composition (e.g., total weight of the article), unless specifically stated otherwise. All ratios are weight ratios, unless specifically stated otherwise. All ranges are inclusive and combinable. The number of significant digits conveys neither a limitation on the indicated amounts nor on the accuracy of the measurements. All numerical amounts are understood to be modified by the word "about" unless otherwise specifically indicated. Unless otherwise indicated, all measurements are understood to be made at 25°C and at ambient conditions, where "ambient conditions" means conditions under about one atmosphere of pressure and at about 50% relative humidity. All such weights as they pertain to listed ingredients are based on the active level and do not include carriers or by-products that may be included in commercially available materials, unless otherwise specified.

"Personal care composition," as used herein, means a composition that may be applied to mammalian keratinous tissue without undue undesirable effects. The personal care composition can be in the form of an article (e.g., porous dissolvable solid substrate), as disclosed herein.

"Keratinous tissue," as used herein, means keratin-containing layers disposed as the outermost protective covering of mammals and includes, but is not limited to, skin, hair, scalp and nails.

The term "non-surfactant cosmetic active" or "cosmetic active" or "benefit agent" or "cosmetic benefit agent" as used herein, means one or a mixture of more than one non-surfactant material(s) that, when applied to mammalian keratinous tissue, provide a benefit to the keratinous tissue. As used herein, the terms "non-surfactant cosmetic active" and "cosmetic active" are used interchangeably. The term "non-surfactant cosmetic active" is broad enough to include skin care actives, hair care actives, and beauty benefit agents, and may be used interchangeably with such terms throughout the present application. Cosmetic actives may deliver beauty benefits such as, but not limited to, sebum inhibition, reducing the oily and/or shiny appearance of skin and/or hair, reducing dryness, itchiness and/or flakiness, reducing skin pore size, exfoliation, desquamation, improving the appearance of the keratinous tissue, conditioning, smoothening, etc.

"Beauty benefit" or "benefit", as used herein in reference to mammalian keratinous tissue includes, but is not limited to cleansing, sebum inhibition, reducing the oily and/or shiny appearance of skin and/or hair, reducing dryness, itchiness and/or flakiness, reducing skin pore size, exfoliation, desquamation, improving the appearance of the keratinous tissue, conditioning, smoothening, etc.

As used herein, the term "conditioning benefit" refers to one or more consumer recognized benefits relating to shine, softness, combability, antistatic properties, wet-handling, damage, manageability, body, and greasiness.

As used herein, "dissolvable" means that the porous dissolvable solid substrate has a dissolution rate that satisfies the Hand Dissolution Method Test described herein.

As used herein "porous dissolvable solid substrate" means a solid, interconnected, polymer-containing matrix that defines a network of spaces or cells that contain the gas of the surrounding atmosphere, typically air. The interconnectivity of the structure may be described by a Star Volume, a Structure Model Index (SMI) or a Percent Open Cell Content.

As used herein, the term "coating" refers to the placement of active(s) onto at least a portion of the surface of the porous dissolvable solid substrate. Due to the porous nature of the substrate, the actives do not spread uniformly across all exposed solid/air interfaces. Rather, the actives of the present invention typically spread, from the point of coating application, into cavities according to gravity.

**Non-Surfactant Hydrophobic Cosmetic Benefit Agent**

The articles of the present invention comprise a hydrophobic surface-resident coating, wherein said coating is or comprises a non-surfactant cosmetic benefit agent. In one embodiment, the article comprises from about 1 % to about 60% hydrophobic surface-resident coating, in other embodiments from about 1% to about 25%, in another embodiment from about 1% to about 15%, and in still another embodiment from about 5% to about 10%.

Any suitable non-surfactant hydrophobic cosmetic benefit agent can be used. For instance, they can include hair or skin conditioners such as silicone, petrolatum, hydrocarbon oils (e.g. mineral oil), natural and synthetic waxes (e.g. micro-crystalline waxes), paraffins, ozokerite, polyethylene, polybutene, polydecene, pentahydrosqualene, vegetable oils, triglycerides, fats, and combinations thereof. Furthermore, the benefit agent can be or can comprise perfume (e.g., perfume oil). Several benefit agents suitable for use herein are described below.

**Conditioning agents**

Conditioning agents include any material which is used to give a particular conditioning benefit to hair and/or skin. In hair treatment compositions, suitable conditioning agents include those which deliver one or more benefits relating to shine, softness, combability, antistatic properties, wet-handling, damage, manageability, body, and greasiness. The conditioning agents useful in the compositions of the present invention typically comprise a water insoluble, and non-volatile liquid. Suitable conditioning agents for use in the composition are those conditioning agents characterized generally as silicones (e.g., silicone oils, cationic silicones, silicone gums, high refractive silicones, and silicone resins), organic conditioning oils (e.g., hydrocarbon oils, polyolefins, and fatty esters) or combinations thereof, or those conditioning agents which otherwise form liquid, dispersed particles in the aqueous surfactant matrix herein.

The concentration of the conditioning agent in the composition should be sufficient to provide the desired conditioning benefits, and as will be apparent to one of ordinary skill in the art. Such concentration can vary with the conditioning agent, the conditioning performance desired, the type and concentration of other components, and other like factors.

**1. Silicones**

The conditioning agent of the compositions of the present invention is preferably an insoluble silicone conditioning agent. The silicone conditioning agent may comprise volatile silicone, non-volatile silicone, or combinations thereof. Preferred are non-volatile silicone conditioning agents. If volatile silicones are present, it will typically be incidental to their use as a solvent or carrier for commercially available forms of non-volatile silicone material ingredients, such as silicone gums and resins. The silicone conditioning agent particles may comprise a silicone fluid conditioning agent and may also comprise other ingredients, such as a silicone resin to improve silicone fluid deposition efficiency or enhance glossiness of the hair.

The concentration of the silicone conditioning agent typically ranges from about 0.5% to about 30%, in one embodiment from about 1% to about 24%, in another embodiment from about 2% to about 16%, and in another embodiment from about 3% to about 8%. Non-limiting examples of suitable silicone conditioning agents, and optional suspending agents for the silicone, are described in U.S. Reissue Pat. No. 34,584, U.S. Pat. No. 5,104,646, and U.S. Pat. No. 5,106,609. The silicone conditioning agents for use in the compositions of the present invention can have a viscosity, as measured at 25°C, of from about 20 to about 2,000,000 centistokes ("csk"), in one embodiment from about 1,000 to about 1,800,000 csk, in other embodiments from about 50,000 to about 1,500,000 csk, and in particular embodiments from about 100,000 to about 1,500,000 csk.

Background material on silicones including sections discussing silicone fluids, gums, and resins, as well as manufacture of silicones, is found in Encyclopedia of Polymer Science and Engineering, vol. 15, 2d ed., pp. 204-308, John Wiley & Sons, Inc. (1989).

The hair conditioning actives of the present invention may comprise one or more silicones including high molecular weight polyalkyl or polyaryl siloxanes and silicone gums; lower molecular weight polydimethyl siloxane fluids; and aminosilicones.

The high molecular weight polyalkyl or polyaryl siloxanes and silicone gums have a viscosity of from about 100,000mPa·s to about 30,000,OOOmPa·s at 25°C, in another embodiment from about 200,000mPa·s to about 30,000,000mPa·s, and a molecular weight of from about 100,000 to about 1,000,000, and in some embodiments from about 120,000 to about 1,000,000.

Preferred higher molecular weight silicone compounds useful herein include polyalkyl or polyaryl siloxanes with the following structure:

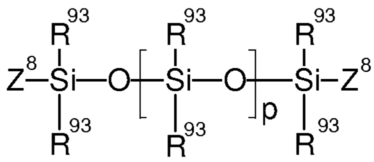

wherein $R^{93}$ is alkyl or aryl, and p is an integer from about 1,300 to about 15,000, more preferably from about 1,600 to about 15,000. $Z^8$ represents groups which block the ends of the silicone chains. The alkyl or aryl groups substituted on the siloxane chain ($R^{93}$) or at the ends of the siloxane chains $Z^8$ can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. Suitable $Z^8$ groups include hydroxy, methyl, methoxy, ethoxy, propoxy, and aryloxy. The two $R^{93}$ groups on the silicon atom may represent the same group or different groups. Preferably, the two $R^{93}$ groups represent the same group. Suitable $R^{93}$ groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. Commercially available silicone compounds useful herein include, for example, those available from the General Electric Company in their TSF451 series, and those available from Dow Corning in their Dow Corning SH200 series.

The silicone compounds that can be used herein can also include a silicone gum. The term "silicone gum", as used herein, means a polyorganosiloxane material having a viscosity at 25°C of greater than or equal to 1,000,000mPa·s. It is recognized that the silicone gums described herein can also have some overlap with the above-disclosed silicone compounds. This overlap is not intended as a limitation on any of these materials. The "silicone gums" will typically have a mass molecular weight in excess of about 165,000, generally between about 165,000 and about 1,000,000. Specific examples include polydimethylsiloxane, poly(dimethylsiloxane methylvinylsiloxane) copolymer, poly(dimethylsiloxane diphenylsiloxane methylvinylsiloxane) copolymer and mixtures thereof. Commercially available silicone gums useful herein include, for example, TSE200A and CF330M available from the General Electric Company.

The lower molecular weight silicones have a viscosity of from about 1mPa·s to about 10,000mPa·s at 25°C, in some embodiments from about 5mPa·s to about 5,000mPa·s, and a molecular weight of from about 400 to about 65,000, and in some embodiments from about 800 to about 50,000.

Preferred lower molecular weight silicone compounds useful herein include polyalkyl or polyaryl siloxanes with the following structure:

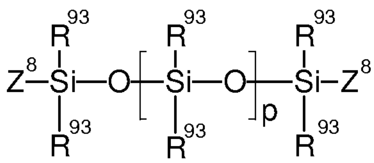

wherein $R^{93}$ is alkyl or aryl, and p is an integer from about 7 to about 850, more preferably from about 7 to about 665. $Z^8$ represents groups which block the ends of the silicone chains. The alkyl or aryl groups substituted on the siloxane chain ($R^{93}$) or at the ends of the siloxane chains $Z^8$ can have any structure as long as the resulting silicone remains fluid at room temperature, is dispersible, is neither irritating, toxic nor otherwise harmful when applied to the hair, is compatible with the other components of the composition, is chemically stable under normal use and storage conditions, and is capable of being deposited on and conditions the hair. Suitable $Z^8$ groups include hydroxy, methyl, methoxy, ethoxy, propoxy, and aryloxy. The two $R^{93}$ groups on the silicon atom may represent the same group or different groups. Preferably, the two $R^{93}$ groups represent the same group. Suitable $R^{93}$ groups include methyl, ethyl, propyl, phenyl, methylphenyl and phenylmethyl. The preferred silicone compounds are polydimethylsiloxane, polydiethylsiloxane, and polymethylphenylsiloxane. Polydimethylsiloxane, which is also known as dimethicone, is especially preferred. Commercially available these silicone compounds useful herein include, for example, those available from the General Electric Company in their TSF451 series, and those available from Dow Corning in their Dow Corning SH200 series.

In one embodiment, the active agent of the present invention includes one or more aminosilicones. Aminosilicones, as provided herein, are silicones containing at least one primary amine, secondary amine, tertiary amine, or a quaternary ammonium group. Preferred aminosilicones may have less than about 0.5% nitrogen by weight of the aminosilicone, more preferably less than about 0.2%, more preferably still, less than about 0.1%. Higher levels of nitrogen (amine functional groups) in the amino silicone tend to result in less friction reduction, and consequently less conditioning benefit from the aminosilicone. It should be understood that in some product forms, higher levels of nitrogen are acceptable

in accordance with the present invention.

In a particular embodiment, the aminosilicone has a viscosity of from about 1,000 centistokes ("csk") to about 100,000 csk, in another embodiment from about 2,000 csk to about 50,000 csk, in yet another embodiment from about 4,000 csk to about 40,000 csk, and in still another embodiment from about 6,000 csk to about 30,000 csk. The viscosity of aminosilicones discussed herein is measured at 25°C.

The aminosilicone can be contained in the composition of the present invention at a level by weight of from about 0.5% to about 30%, in an alternate embodiment from about 1.0% to about 24%, in another embodiment from about 2.0% to about 16%, and in yet another embodiment from about 3.0% to about 8%.

Examples of preferred aminosilicones for use in embodiments of the subject invention include, but are not limited to, those which conform to the general formula (I):

$$(R^1)_aG_{3-a}\text{-Si-(-OSiG}_2)_n\text{-(-OSiG}_b(R^1)_{2-b})_m\text{-O-SiG}_{3-a}(R^1)_a \qquad \text{(I)}$$

wherein G is hydrogen, phenyl, hydroxy, or $C_1$-$C_8$ alkyl, preferably methyl; a is 0 or an integer having a value from 1 to 3, preferably 1; b is 0, 1, or 2, preferably 1; wherein when a is 0, b is not 2; n is a number from 0 to 1,999; m is an integer from 0 to 1,999; the sum of n and m is a number from 1 to 2,000; a and m are not both 0; $R^1$ is a monovalent radical conforming to the general formula $CqH_{2q}L$, wherein q is an integer having a value from 2 to 8 and L is selected from the following groups: $-N(R^2)CH_2-CH_2-N(R^2)_2$; $-N(R^2)_2$; $-N(R^2)^+{}_3A^-$; $-N(R^2)CH_2-CH_2-N\ R^2H_2A^-$; wherein $R^2$ is hydrogen, phenyl, benzyl, or a saturated hydrocarbon radical, preferably an alkyl radical from about $C_1$ to about $C_{20}$; A is a halide ion.

Some silicones for use herein can include those aminosilicones that correspond to formula (I) wherein m=0, a=1, q=3, G=methyl, n is preferably from about 1500 to about 1700, more preferably about 1600; and L is $-N(CH_3)_2$ or $-NH_2$, more preferably $-NH_2$. Other aminosilicones can include those corresponding to formula (I) wherein m=0, a=1, q=3, G=methyl, n is preferably from about 400 to about 600, more preferably about 500; and L is$-N(CH_3)_2$ or $-NH_2$, more preferably $-NH_2$. These aminosilicones can be called as terminal aminosilicones, as one or both ends of the silicone chain are terminated by nitrogen containing group.

An exemplary aminosilicone corresponding to formula (I) is the polymer known as "trimethylsilylamodimethicone", which is shown below in formula (II):

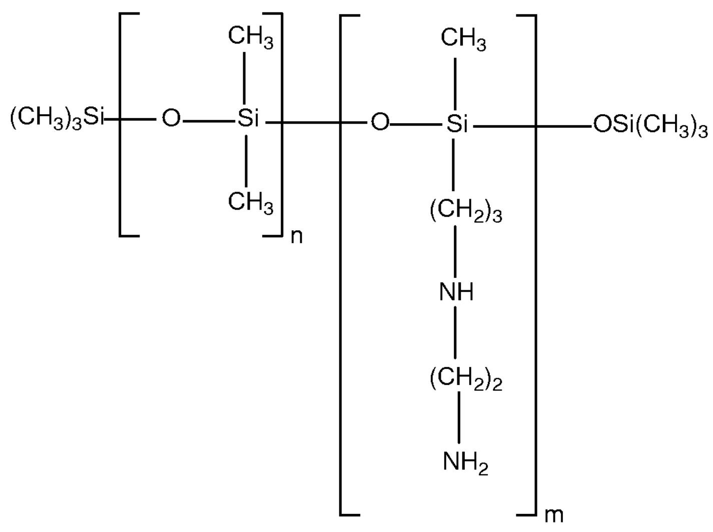

(II)

wherein n is a number from 1 to 1,999 and m is a number from 1 to 1,999.

## 2. Organic conditioning oils

The conditioning component of the compositions of the present invention may also comprise from about 0.05% to about 3%, in one embodiment from about 0.08% to about 1.5%, and in a particular embodiment from about 0.1 % to about 1%, of at least one organic conditioning oil as the conditioning agent, either alone or in combination with other conditioning agents, such as the silicones.

Hydrocarbon based benefit materials suitable in the present invention can have a Young's Modulus between 100 to 2,000 Pa. In one embodiment, the hydrocarbon based benefit material comprises an average carbon chain length

of greater than 20, in another embodiment an average carbon chain length of greater than 30, and in still other embodiments an average carbon chain length of greater than 40.

The hydrocarbon based benefit materials have a preferred rheology profile as defined by Consistency value (k) and Shear Index (n). The term "Consistency value" or "k" as used herein is a measure of lipid viscosity and is used in combination with Shear Index, to define viscosity for materials whose viscosity is a function of shear. The measurements are made at 35°C and the units are poise (equal to 100 cps). The term "Shear Index" or "n" as used herein is a measure of lipid viscosity and is used in combination with Consistency value, to define viscosity for materials whose viscosity is a function of shear. The measurements are made at 35°C and the units are dimensionless. Consistency value (k) and Shear Index (n) are more fully described in the Test Methods below. Preferred Consistency value ranges are 1-10,000 poise $(1/sec)^{n-1}$, preferably 10-2000 poise $(1/sec)^{n-1}$ and more preferably 50-1000 poise $(1/sec)^{n-1}$. Shear Index ranges are 0.1-0.8, preferably 0.1-0.5 and more preferably 0.20-0.4. These preferred rheological properties are especially useful in providing compositions with improved deposition of benefit agents on skin.

**a**. **Hydrocarbon oils**

Suitable organic conditioning oils for use as conditioning agents in the compositions of the present invention include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils preferably are from about $C_{12}$ to about $C_{19}$. Branched chain hydrocarbon oils, including hydrocarbon polymers, typically will contain more than 19 carbon atoms.

Specific non-limiting examples of these hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from Amoco Chemical Corporation. The concentration of such hydrocarbon oils in the composition can range from about 0.05% to about 20%, alternatively from about 0.08% to about 1.5%, and alternatively from about 0.1 % to about 1%.

**b**. **Polyolefins**

Organic conditioning oils for use in the compositions of the present invention can also include liquid polyolefins, more preferably liquid poly-α-olefins, more preferably hydrogenated liquid poly-α-olefins. Polyolefins for use herein are prepared by polymerization of $C_4$ to about $C_{14}$ olefenic monomers, preferably from about $C_6$ to about $C_{12}$.

Non-limiting examples of olefenic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene, and mixtures thereof. Also suitable for preparing the polyolefin liquids are olefin-containing refinery feedstocks or effluents. Preferred hydrogenated α-olefin monomers include, but are not limited to: 1-hexene to 1-hexadecenes, 1-octene to 1-tetradecene, and mixtures thereof.

**c. Fatty Esters**

Other suitable organic conditioning oils for use as the conditioning agent in the compositions of the present invention include, but are not limited to, fatty esters having at least 10 carbon atoms. These fatty esters include esters with hydrocarbyl chains derived from fatty acids or alcohols (e.g. mono-esters, polyhydric alcohol esters, and di- and tri-carboxylic acid esters). The hydrocarbyl radicals of the fatty esters hereof may include or have covalently bonded thereto other compatible functionalities, such as amides and alkoxy moieties (e.g., ethoxy or ether linkages, etc.).

Specific examples of preferred fatty esters include, but are not limited to: isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Other fatty esters suitable for use in the compositions of the present invention are monocarboxylic acid esters of the general formula R'COOR, wherein R' and R are alkyl or alkenyl radicals, and the sum of carbon atoms in R' and R is at least 10, preferably at least 22.

Still other fatty esters suitable for use in the compositions of the present invention are di-and tri-alkyl and alkenyl esters of carboxylic acids, such as esters of $C_4$ to $C_8$ dicarboxylic acids (e.g. $C_1$ to $C_{22}$ esters, preferably $C_1$ to $C_6$, of succinic acid, glutaric acid, and adipic acid). Specific non-limiting examples of di- and tri- alkyl and alkenyl esters of carboxylic acids include isocetyl stearyol stearate, diisopropyl adipate, and tristearyl citrate.

Other fatty esters suitable for use in the compositions of the present invention are those known as polyhydric alcohol esters. Such polyhydric alcohol esters include alkylene glycol esters, such as ethylene glycol mono and di-fatty acids, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.

Still other fatty esters suitable for use in the compositions of the present invention are glycerides, including, but not limited to, mono-, di-, and tri-glycerides, preferably di- and tri-glycerides, more preferably triglycerides. For use in the compositions described herein, the glycerides are preferably the mono-, di-, and tri-esters of glycerol and long chain carboxylic acids, such as $C_{10}$ to $C_{22}$ carboxylic acids. A variety of these types of materials can be obtained from vegetable and animal fats and oils, such as castor oil, safflower oil, cottonseed oil, corn oil, olive oil, cod liver oil, almond oil, avocado oil, palm oil, sesame oil, lanolin and soybean oil. Synthetic oils include, but are not limited to, triolein and tristearin glyceryl dilaurate.

Other fatty esters suitable for use in the compositions of the present invention are water insoluble synthetic fatty esters. Some preferred synthetic esters conform to the general Formula (IX):

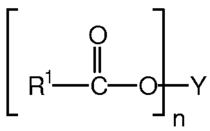

wherein $R^1$ is a $C_7$ to $C_9$ alkyl, alkenyl, hydroxyalkyl or hydroxyalkenyl group, preferably a saturated alkyl group, more preferably a saturated, linear, alkyl group; n is a positive integer having a value from 2 to 4, preferably 3; and Y is an alkyl, alkenyl, hydroxy or carboxy substituted alkyl or alkenyl, having from about 2 to about 20 carbon atoms, preferably from about 3 to about 14 carbon atoms. Other preferred synthetic esters conform to the general Formula (X):

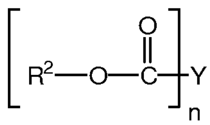

wherein $R^2$ is a $C_8$ to $C_{10}$ alkyl, alkenyl, hydroxyalkyl or hydroxyalkenyl group; preferably a saturated alkyl group, more preferably a saturated, linear, alkyl group; n and Y are as defined above in Formula (X).

Specific non-limiting examples of suitable synthetic fatty esters for use in the compositions of the present invention include: P-43 ($C_8$-$C_{10}$ triester of trimethylolpropane), MCP-684 (tetraester of 3,3 diethanol-1,5 pentadiol), MCP 121 ($C_8$-$C_{10}$ diester of adipic acid), all of which are available from Mobil Chemical Company.

**3. Other conditioning agents**

Also suitable for use in the compositions herein are the conditioning agents described by the Procter & Gamble Company in U.S. Pat. Nos. 5,674,478, and 5,750,122. Also suitable for use herein are those conditioning agents described in U.S. Pat. Nos. 4,529,586 (Clairol), 4,507,280 (Clairol), 4,663,158 (Clairol), 4,197,865 (L'Oreal), 4,217, 914 (L'Oreal), 4,381,919 (L'Oreal), and 4,422, 853 (L'Oreal).

**Perfumes**

The non-surfactant hydrophobic cosmetic benefit agent of the present invention may also include one or more perfumes. The one or more perfumes may be selected from any perfume or perfume chemical suitable for topical application to the skin and/or hair and suitable for use in personal care compositions. The concentration of the perfume in the personal care composition should be effective to provide the desired aroma including, but not limited to, unscented. Generally, the concentration of the scented primary perfume is from about 0.5% to about 30%, in one embodiment from about 1% to about 20%, in yet another embodiment from about 2% to about 10%, and in yet another embodiment from

about 3% to about 8%, by weight of the solid article.

The perfume may be selected from the group consisting of perfumes, highly volatile perfume materials having a boiling point of less than about 250°C, and mixtures thereof. In one embodiment, the perfume is selected from high impact accord perfume ingredients having a ClogP of greater than about 2 and odor detection thresholds of less than or equal to 50 parts per billion (ppb).

## Optional Components

The compositions of the present invention may also contain any suitable optional components. Optional components can include, for example, vitamins and amino acids such as: water soluble vitamins such as vitamin B1, B2, B6, B12, C, pantothenic acid, pantothenyl ethyl ether, panthenol, biotin, and their derivatives, water soluble amino acids such as asparagine, alanin, indole, glutamic acid and their salts, water insoluble vitamins such as vitamin A, D, E, and their derivatives, water insoluble amino acids such as tyrosine, tryptamine, and their salts.

The compositions of the present invention may also contain pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C. I. Names. The compositions of the present invention may also contain antimicrobial agents which are useful as cosmetic biocides and antidandruff agents including: water soluble components such as piroctone olamine, water insoluble components such as 3,4,4'- trichlorocarbanilide (trichlosan), triclocarban and zinc pyrithione. The compositions of the present invention may also contain chelating agents. Several optional components are described in more detail below.

## Anti-dandruff Actives

The compositions of the present invention may also contain an anti-dandruff agent. Suitable, non-limiting examples of anti-dandruff particulates include: pyridinethione salts, azoles, selenium sulfide, particulate sulfur, and mixtures thereof. Preferred are pyridinethione salts. Such anti-dandruff particulate should be physically and chemically compatible with the essential components of the composition, and should not otherwise unduly impair product stability, aesthetics or performance.

### 1. Pyridinethione salts

Pyridinethione anti-dandruff particulates, especially 1-hydroxy-2-pyridinethione salts, are highly preferred particulate anti-dandruff agents for use in compositions of the present invention. The concentration of pyridinethione anti-dandruff particulate typically ranges from about 0.1% to about 4%, by weight of the composition, preferably from about 0.1% to about 3%, more preferably from about 0.3% to about 2%. Preferred pyridinethione salts include those formed from heavy metals such as zinc, tin, cadmium, magnesium, aluminum and zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form, wherein the particles have an average size of up to about 20μ, preferably up to about 5μ, more preferably up to about 2.5μ. Salts formed from other cations, such as sodium, may also be suitable. Pyridinethione anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982. It is contemplated that when ZPT is used as the anti-dandruff particulate in the compositions herein, that the growth or re-growth of hair may be stimulated or regulated, or both, or that hair loss may be reduced or inhibited, or that hair may appear thicker or fuller.

### 2. Other Anti-microbial Actives

In addition to the anti-dandruff active selected from polyvalent metal salts of pyrithione, the present invention may further comprise one or more anti-fungal or anti-microbial actives in addition to the metal pyrithione salt actives. Suitable anti-microbial actives include coal tar, sulfur, whitfield's ointment, castellani's paint, aluminum chloride, gentian violet, octopirox (piroctone olamine), ciclopirox olamine, undecylenic acid and it's metal salts, potassium permanganate, selenium sulphide, sodium thiosulfate, propylene glycol, oil of bitter orange, urea preparations, griseofulvin, 8-Hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone and azoles, and combinations thereof. Preferred anti-microbials include itraconazole, ketoconazole, selenium sulphide and coal tar.

a. **Azoles**

Azole anti-microbials include imidazoles such as benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole and itraconazole, and combinations thereof. When present in the composition, the azole anti-microbial active is included in an amount from about 0.01% to about 5%, preferably from about 0.1% to about 3%, and more preferably from about 0.3% to about 2%, by weight of the composition. Especially preferred herein is ketoconazole.

b. **Selenium Sulfide**

Selenium sulfide is a particulate anti-dandruff agent suitable for use in the anti-microbial compositions of the present invention, effective concentrations of which range from about 0.1% to about 4%, by weight of the composition, preferably from about 0.3% to about 2.5%, more preferably from about 0.5% to about 1.5%. Selenium sulfide is generally regarded as a compound having one mole of selenium and two moles of sulfur, although it may also be a cyclic structure that conforms to the general formula $Se_xS_y$, wherein x + y = 8. Average particle diameters for the selenium sulfide are typically less than 15μm, as measured by forward laser light scattering device (e.g. Malvern 3600 instrument), preferably less than 10 μm. Selenium sulfide compounds are described, for example, in U.S. Pat. No. 2,694,668; U.S. Pat. No. 3,152,046; U.S. Pat. No. 4,089,945; and U.S. Pat. No. 4,885,107.

c. **Sulfur**

Sulfur may also be used as a particulate anti-microbial/anti-dandruff agent in the anti-microbial compositions of the present invention. Effective concentrations of the particulate sulfur are typically from about 1% to about 4%, by weight of the composition, preferably from about 2% to about 4%.

d. **Keratolytic Agents**

The present invention may further comprise one or more keratolytic agents such as Salicylic Acid.

e. **Additional Anti-microbial Actives**

Additional anti-microbial actives of the present invention may include extracts of melaleuca (tea tree) and charcoal. The present invention may also comprise combinations of anti-microbial actives. Such combinations may include octopirox and zinc pyrithione combinations, pine tar and sulfur combinations, salicylic acid and zinc pyrithione combinations, octopirox and climbasole combinations, and salicylic acid and octopirox combinations, and mixtures thereof. These actives, when used herein, are used at levels of from about 1% to about 4%, preferably from about 2% to about 4%.

**Surfactants**

The dissolvable personal care solids of the present invention comprise one or more surfactants suitable for application to the hair or skin. Surfactants suitable for use in the dissolvable porous solids of the present invention include anionic surfactants, nonionic surfactants, cationic surfactants, zwitterionic surfactants, amphoteric surfactants, or combinations thereof. The surfactant component of the dissolvable porous solid is essential in preparing a stable structure for the dissolvable porous solids described herein, although it is understood that the surfactant component can also be used to provide in addition to stable solid porous structure, a cleansing material for use in the method of the present invention. Likewise, the surfactant component can also be used solely or primarily as a process aid in making a stable foam, wherein the surfactant includes conventional surfactants or emulsifiers that need not provide any lathering performance. Examples of emulsifiers for use as a surfactant component herein include mono-and di-glycerides, fatty alcohols, polyglycerol esters, propylene glycol esters, sorbitan esters and other emulsifiers known or otherwise commonly used to stabilized air interfaces, as for example those used during preparation of aerated foodstuffs such as cakes and other baked goods and confectionary products, or the stabilization of cosmetics such as hair mousses.

In one embodiment, the article is a substantially non-lathering dissolvable solid personal care product and comprises from about 0% to about 10% by weight of the article of an ionic (e.g., anionic, zwitterionic, cationic or mixtures thereof) surfactant, in one embodiment from about 0% to about 5% by weight of the article of an ionic surfactant, and in one embodiment from about 0% to about 2.5% by weight of the article of an ionic surfactant, and from about 1% to

about 50% by weight of the article of a nonionic or polymeric surfactant, in one embodiment from about 5% to about 45% by weight of the article of a nonionic or polymeric surfactant, and in one embodiment from about 10% to about 40% by weight of the article of a nonionic or polymeric surfactant, and combinations thereof.

In another embodiment, the article is a lathering dissolvable solid personal care product that comprises from about 23% to about 75% ionic surfactant, in one embodiment from about 30% to about 70%, and in another embodiment from about 50% to about 65% ionic surfactant.

Anionic surfactants suitable for use in the personal care compositions of the present invention include those described in McCutcheon's Detergents and Emulsifiers, North American Edition (1986), Allured Publishing Corp.; McCutcheon's, Functional Materials, North American Edition (1992), Allured Publishing Corp.; and U.S. Patent 3,929,678 (Laughlin et al.), which descriptions are incorporated herein by reference.

Non-limiting examples of anionic surfactants suitable for use herein include alkyl and alkyl ether sulfates, sulfated monoglycerides, sulfonated olefins, alkyl aryl sulfonates, primary or secondary alkane sulfonates, alkyl sulfosuccinates, acyl taurates, acyl isethionates, alkyl glycerylether sulfonate, sulfonated methyl esters, sulfonated fatty acids, alkyl phosphates, acyl glutamates, acyl sarcosinates, alkyl sulfoacetates, acylated peptides, alkyl ether carboxylates, acyl lactylates, anionic fluorosurfactants, sodium lauroyl glutamate, and combinations therof.

Anionic surfactants suitable for use in the personal care compositions of the present invention include alkyl and alkyl ether sulfates. These materials have the respective formulae $ROSO_3M$ and $RO(C_2H_4O)_xSO_3M$, wherein R is alkyl or alkenyl of from about 8 to about 24 carbon atoms, x is 1 to 10, and M is a water-soluble cation such as ammonium, sodium, potassium and triethanolamine. The alkyl ether sulfates are typically made as condensation products of ethylene oxide and monohydric alcohol's having from about 8 to about 24 carbon atoms. Preferably, R has from about 10 to about 18 carbon atoms in both the alkyl and alkyl ether sulfates. The alcohol's can be derived from fats, e.g., coconut oil or tallow, or can be synthetic. Lauryl alcohol and straight chain alcohol's derived from coconut oil are preferred herein. Such alcohol's are reacted with about 1 to about 10, preferably from about 3 to about 5, and especially about 3, molar proportions of ethylene oxide and the resulting mixture of molecular species having, for example, an average of 3 moles of ethylene oxide per mole of alcohol, is sulfated and neutralized.

Specific examples of alkyl ether sulfates which may be used in the personal care compositions are sodium and ammonium salts of coconut alkyl triethylene glycol ether sulfate; tallow alkyl triethylene glycol ether sulfate, and tallow alkyl hexaoxyethylene sulfate. Highly preferred alkyl ether sulfates are those comprising a mixture of individual compounds, said mixture having an average alkyl chain length of from about 10 to about 16 carbon atoms and an average degree of ethoxylation of from about 1 to about 4 moles of ethylene oxide.

Other suitable anionic surfactants include water-soluble salts of the organic, sulfuric acid reaction products of the general formula $[R^1\text{-}SO_3\text{-}M]$, wherein $R^1$ is chosen from the group consisting of a straight or branched chain, saturated aliphatic hydrocarbon radical having from about 8 to about 24, preferably about 10 to about 18, carbon atoms; and M is a cation. Important examples are the salts of an organic sulfuric acid reaction product of a hydrocarbon of the methane series, including iso-, neo-, ineso-, and n-paraffins, having about 8 to about 24 carbon atoms, preferably about 10 to about 18 carbon atoms and a sulfonating agent, e.g., $SO_3$, $H_2SO_4$, oleum, obtained according to known sulfonation methods, including bleaching and hydrolysis. Preferred are alkali metal and ammonium sulfonated $C_{10\text{-}18}$ n-paraffins.

Additional examples of suitable anionic surfactants are the reaction products of fatty acids esterified with isethionic acid and neutralized with sodium hydroxide where, for example, the fatty acids are derived from coconut oil; sodium or potassium salts of fatty acid amides of methyl tauride in which the fatty acids, for example, are derived from coconut oil. Other suitable anionic surfactants of this variety are described in U.S. Patent 2,486,921, U.S. Patent 2,486,922 and U.S. Patent 2,396,278.

Still other suitable anionic surfactants are the succinamates, examples of which include disodium N-octadecylsulfosuccinamate; diammoniumlauryl sulfosuccinamate; tetrasodium N-(1,2-dicarboxyethyl)-N-octadecylsulfosuccinamate; diamyl ester of sodium sulfosuccinic acid; dihexyl ester of sodium sulfosuccinic acid; and dioctyl esters of sodium sulfosuccinic acid.

Other suitable anionic surfactants include olefin sulfonates having about 12 to about 24 carbon atoms. The term "olefin sulfonates" is used herein to mean compounds which can be produced by the sulfonation of a-olefins by means of uncomplexed sulfur trioxide, followed by neutralization of the acid reaction mixture in conditions such that any sulfones which have been formed in the reaction are hydrolyzed to give the corresponding hydroxy-alkanesulfonates. The sulfur trioxide can be liquid or gaseous, and is usually, but not necessarily, diluted by inert diluents, for example by liquid $SO_2$, chlorinated hydrocarbons, etc., when used in the liquid form, or by air, nitrogen, gaseous $SO_2$, etc., when used in the gaseous form.

The alpha-olefins from which the olefin sulfonates are derived are mono-olefins having about 12 to about 24 carbon atoms, preferably about 14 to about 16 carbon atoms. Preferably, they are straight chain olefins.

In addition to the true alkene sulfonates and a proportion of hydroxy-alkanesulfonates, the olefin sulfonates can contain minor amounts of other materials, such as alkene disulfonates depending upon the reaction conditions, proportion of reactants, the nature of the starting olefins and impurities in the olefin stock and side reactions during the

sulfonation process.

Another class of anionic surfactants suitable for use in the personal care compositions are the b-alkyloxy alkane sulfonates. These compounds have the following formula:

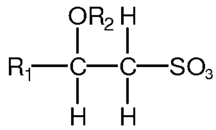

where $R_1$ is a straight chain alkyl group having from about 6 to about 20 carbon atoms, $R_2$ is a lower alkyl group having from about 1 (preferred) to about 3 carbon atoms, and M is a water-soluble cation as hereinbefore described.

Other suitable surfactants are described in McCutcheon's, Emulsifiers and Detergents, 1989 Annual, published by M. C. Publishing Co., and in U.S. Patent 3,929,678, which descriptions are incorporated herein by reference.

Preferred anionic surfactants for use in the personal care compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, and combinations thereof.

Amphoteric surfactants suitable for use in the personal care compositions of the present invention includes those that are broadly described as derivatives of aliphatic secondary and tertiary amines in which the aliphatic radical can be straight or branched chain and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecyl-aminopropionate, sodium 3-dodecylaminopropane sulfonate, sodium lauryl sarcosinate, N-alkyltaurines such as the one prepared by reacting dodecylamine with sodium isethionate according to the teaching of U.S. Patent 2,658,072, N-higher alkyl aspartic acids such as those produced according to the teaching of U.S. Patent 2,438,091, and the products described in U.S. Patent 2,528,378. Zwitterionic surfactants suitable for use include those that are broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate. Zwitterionic surfactants suitable for use in the multiphase, personal care composition include betaines, including cocoamidopropyl betaine.

The amphoteric surfactants of the present invention may also include alkylamphoacetates including lauroamphoacetate and cocoamphoacetate. Alkylamphoacetates can be comprised of monoacetates and diacetates. In some types of alkylamphoacetates, diacetates are impurities or unintended reaction products.

Cationic surfactants can also be used as cleansing agents in the personal care compositions of the present invention, but are generally less preferred, and preferably represent less than about 5% by weight of the compositions.

Suitable nonionic surfactants for use as lathering surfactants in the personal care compositions of the present invention include those described in McCutcheion's Detergents and Emulsifiers, North American edition (1986), Allured Publishing Corp., and McCutcheion's Functional Materials, North American edition (1992), which descriptions are incorporated herein by reference. These nonionic lathering surfactants suitable for use herein include alkyl glucosides, alkyl polyglucosides, polyhydroxy fatty acid amides, alkoxylated fatty acid esters, sucrose esters, amine oxides, and combinations thereof.

Suitable nonionic surfactants for use in the personal care compositions of the present invention include condensation products of alkylene oxide groups (hydrophilic in nature) with an organic hydrophobic compound, which may be aliphatic or alkyl aromatic in nature. Preferred classes of nonionic surfactants include:

1) polyethylene oxide condensates of alkyl phenols, e.g., the condensation products of alkyl phenols having an alkyl group containing from about 6 to about 20 carbon atoms in either a straight chain or branched chain configuration, with ethylene oxide, the ethylene oxide being present in amounts equal to from about 10 to about 60 moles of ethylene oxide per mole of alkyl phenol;
2) nonionic surfactants derived from the condensation of ethylene oxide with the product resulting from the reaction of propylene oxide and ethylene diamine products;
3) condensation products of aliphatic alcohol's having from about 8 to about 18 carbon atoms, in either straight

chain or branched chain configuration, with ethylene oxide, e.g., a coconut alcohol ethylene oxide condensate having from about 10 to about 30 moles of ethylene oxide per mole of coconut alcohol, the coconut alcohol fraction having from about 10 to about 14 carbon atoms;

4) long chain tertiary amine oxides corresponding to the following general formula:

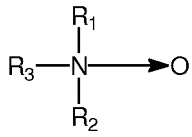

wherein $R_1$ contains an alkyl, alkenyl or monohydroxy alkyl radical of from about 8 to about 18 carbon atoms, from 0 to about 10 ethylene oxide moieties, and from 0 to about 1 glyceryl moiety, and $R_2$ and $R_3$ contain from about 1 to about 3 carbon atoms and from 0 to about 1 hydroxy group, e.g., methyl, ethyl, propyl, hydroxyethyl, or hydroxypropyl radicals;

5) long chain tertiary phosphine oxides corresponding to the following general formula:

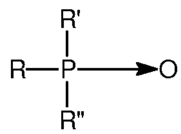

wherein R contains an alkyl, alkenyl or monohydroxyalkyl radical ranging from about 8 to about 18 carbon atoms in chain length, from 0 to about 10 ethylene oxide moieties and from 0 to about 1 glyceryl moiety and R' and R" are each alkyl or monohydroxyalkyl groups containing from about 1 to about 3 carbon atoms;

6) long chain dialkyl sulfoxides containing one short chain alkyl or hydroxy alkyl radical of from about 1 to about 3 carbon atoms (usually methyl) and one long hydrophobic chain which include alkyl, alkenyl, hydroxy alkyl, or keto alkyl radicals containing from about 8 to about 20 carbon atoms, from 0 to about 10 ethylene oxide moieties and from 0 to about 1 glyceryl moiety;

7) alkyl polysaccharide (APS) surfactants such as the alkyl polyglycosides, as described in U.S. Patent 4,565,647, which have a hydrophobic group with about 6 to about 30 carbon atoms and polysaccharide (e.g., polyglycoside) as the hydrophilic group, and optionally have a polyalkylene-oxide group joining the hydrophobic and hydrophilic moieties, wherein the alkyl group (i.e., the hydrophobic moiety) can be saturated or unsaturated, branched or unbranched, and unsubstituted or substituted (e.g., with hydroxy or cyclic rings); and

8) polyethylene glycol (PEG) glyceryl fatty esters, such as those of the formula $R(O)OCH_2CH(OH)CH_2(OCH_2CH_2)_nOH$ wherein n is from about 5 to about 200, preferably from about 20 to about 100, and R is an aliphatic hydrocarbyl having from about 8 to about 20 carbon atoms.

Zwitterionic surfactants suitable for use in the personal care compositions of the present invention include those that are broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group, e.g., carboxy, sulfonate, sulfate, phosphate, or phosphonate. Such suitable zwitterionic surfactants can be represented by the formula:

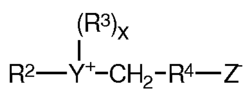

wherein $R^2$ contains an alkyl, alkenyl, or hydroxy alkyl radical of from about 8 to about 18 carbon atoms, from 0 to about 10 ethylene oxide moieties and from 0 to about 1 glyceryl moiety; Y is selected from the group consisting of nitrogen, phosphorus, and sulfur atoms; $R^3$ is an alkyl or monohydroxyalkyl group containing about 1 to about 3 carbon atoms; X is 1 when Y is a sulfur atom, and 2 when Y is a nitrogen or phosphorus atom; $R^4$ is an alkylene or hydroxyalkylene of from about 1 to about 4 carbon atoms and Z is a radical selected from the group consisting of carboxylate, sulfonate, sulfate, phosphonate, and phosphate groups.

Other zwitterionic surfactants suitable for use herein include betaines, including high alkyl betaines such as coco dimethyl carboxymethyl betaine, cocoamidopropyl betaine, cocobetaine, lauryl amidopropyl betaine, oleyl betaine,

lauryl dimethyl carboxymethyl betaine, lauryl dimethyl alphacarboxyethyl betaine, cetyl dimethyl carboxymethyl betaine, lauryl bis-(2-hydroxyethyl) carboxymethyl betaine, stearyl bis-(2-hydroxypropyl) carboxymethyl betaine, oleyl dimethyl gamma-carboxypropyl betaine, and lauryl bis-(2-hydroxypropyl)alphacarboxyethyl betaine. The sulfobetaines may be represented by coco dimethyl sulfopropyl betaine, stearyl dimethyl sulfopropyl betaine, lauryl dimethyl sulfoethyl betaine, lauryl bis-(2-hydroxyethyl) sulfopropyl betaine and the like; amidobetaines and amidosulfobetaines, wherein the $RCONH(CH_2)_3$ radical is attached to the nitrogen atom of the betaine are also useful in this invention.

Suitable nonionic surfactants for use in the present invention include those described in McCutcheion's Detergents and Emulsifiers, North American edition (1986), Allured Publishing Corp., and McCutcheion's Functional Materials, North American edition (1992). Suitable nonionic surfactants for use in the personal care compositions of the present invention include, but are not limited to, polyoxyethylenated alkyl phenols, polyoxyethylenated alcohols, polyoxyethylenated polyoxypropylene glycols, glyceryl esters of alkanoic acids, polyglyceryl esters of alkanoic acids, propylene glycol esters of alkanoic acids, sorbitol esters of alkanoic acids, polyoxyethylenated sorbitor esters of alkanoic acids, polyoxyethylene glycol esters of alkanoic acids, polyoxyethylenated alkanoic acids, alkanolamides, N-alkylpyrrolidones, alkyl glycosides, alkyl polyglucosides, alkylamine oxides, and polyoxyethylenated silicones.

In a particular embodiment, the nonionic surfactant selected from sorbitan esters and alkoxylated derivatives of sorbitan esters including sorbitan monolaurate (SPAN® 20), sorbitan monopalmitate (SPAN® 40), sorbitan monostearate (SPAN® 60), sorbitan tristearate (SPAN@ 65), sorbitan monooleate (SPAN® 80), sorbitan trioleate (SPAN® 85), sorbitan isostearate, polyoxyethylene (20) sorbitan monolaurate (Tween® 20), polyoxyethylene (20) sorbitan monopalmitate (Tween® 40), polyoxyethylene (20) sorbitan monostearate (Tween® 60), polyoxyethylene (20) sorbitan monooleate (Tween® 80), polyoxyethylene (4) sorbitan monolaurate (Tween® 21), polyoxyethylene (4) sorbitan monostearate (Tween® 61), polyoxyethylene (5) sorbitan monooleate (Tween® 81), all available from Uniqema, and combinations thereof.

Suitable polymeric surfactants for use in the personal care compositions of the present invention include, but are not limited to, block copolymers of ethylene oxide and fatty alkyl residues, block copolymers of ethylene oxide and propylene oxide, hydrophobically modified polyacrylates, hydrophobically modified celluloses, silicone polyethers, silicone copolyol esters, diquaternary polydimethylsiloxanes, and co-modified amino/polyether silicones

**<u>Water-Soluble Polymer ("Polymer Structurant")</u>**

The present invention comprises water-soluble polymer that functions as a structurant. In one embodiment the article is non-lathering and comprises from about 15% to about 70% polymeric water soluble polymer, in another embodiment from about 20% to about 60%, and in still another embodiment from about 25% to about 50%. In another embodiment the article is lathering and comprises from about 10% to about 50% water soluble polymer, in another embodiment from about 15% to about 40%, and in yet another embodiment from about 20% to about 30%.

As used herein, the term "water-soluble polymer" is broad enough to include both water-soluble and water-dispersible polymers, and is defined as a polymer with a solubility in water, measured at 25°C, of at least about 0.1 gram/liter (g/L). In some embodiments, the polymers have a solubility in water, measured at 25°C, of from about 0.1 gram/liter (g/L).to about 500 grams/liter (g/L). This indicates production of a macroscopically isotropic or transparent, colored or colorless solution. The polymers for making these solids may be of synthetic or natural origin and may be modified by means of chemical reactions. They may or may not be film-forming. These polymers should be physiologically acceptable, i.e., they should be compatible with the skin, mucous membranes, the hair and the scalp.

The terms "water-soluble polymer" and "polymer structurant" are used interchangeably herein. Furthermore, whenever the singular term "polymer" is stated, it should be understood that the term is broad enough to include one polymer or a mixture of more than one polymer. For instance, if a mixture of polymers is used, the polymer solubility as referred to herein would refer to the solubility of the mixture of polymers, rather than to the solubility of each polymer individually.

The one or more water-soluble polymers of the present invention are selected such that their weighted average molecular weight is from about 40,000 to about 500,000, in one embodiment from about 50,000 to about 400,000, in yet another embodiment from about 60,000 to about 300,000, and in still another embodiment from about 70,000 to about 200,000. The weighted average molecular weight is computed by summing the average molecular weights of each polymer raw material multiplied by their respective relative weight percentages by weight of the total weight of polymers present within the porous solid.

The water-soluble polymer(s) of the present invention can include, but are not limited to, synthetic polymers including polyvinyl alcohols, polyvinylpyrrolidones, polyalkylene oxides, polyacrylates, caprolactams, polymethacrylates, polymethylmethacrylates, polyacrylamides, polymethylacrylamides, polydimethylacrylamides, polyethylene glycol monomethacrylates, polyurethanes, polycarboxylic acids, polyvinyl acetates, polyesters, polyamides, polyamines, polyethyleneimines, maleic/(acrylate or methacrylate) copolymers, copolymers of methylvinyl ether and of maleic anhydride, copolymers of vinyl acetate and crotonic acid, copolymers of vinylpyrrolidone and of vinyl acetate, copolymers of vinylpyrrolidone and of caprolactam, vinyl pyrollidone/vinyl acetate copolymers, copolymers of anionic, cationic and amphoteric monomers, and combinations thereof.

The water-soluble polymer(s) of the present invention may also be selected from naturally sourced polymers including those of plant origin examples of which include karaya gum, tragacanth gum, gum Arabic, acemannan, konjac mannan, acacia gum, gum ghatti, whey protein isolate, and soy protein isolate; seed extracts including guar gum, locust bean gum, quince seed, and psyllium seed; seaweed extracts such as Carrageenan, alginates, and agar; fruit extracts (pectins); those of microbial origin including xanthan gum, gellan gum, pullulan, hyaluronic acid, chondroitin sulfate, and dextran; and those of animal origin including casein, gelatin, keratin, keratin hydrolysates, sulfonic keratins, albumin, collagen, glutelin, glucagons, gluten, zein, and shellac.

Modified natural polymers are also useful as water-soluble polymer(s) in the present invention. Suitable modified natural polymers include, but are not limited to, cellulose derivatives such as hydroxypropylmethylcellulose, hydroxymethylcellulose, hydroxyethylcellulose, methylcellulose, hydroxypropylcellulose, ethylcellulose, carboxymethylcellulose, cellulose acetate phthalate, nitrocellulose and other cellulose ethers/esters; and guar derivatives such as hydroxypropyl guar.

Preferred water-soluble polymers of the present invention include polyvinyl alcohols, polyvinylpyrrolidones, polyalkylene oxides, starch and starch derivatives, pullulan, gelatin, hydroxypropylmethylcelluloses, methycelluloses, and carboxymethycelluloses.

More preferred water-soluble polymers of the present invention include polyvinyl alcohols, and hydroxypropylmethylcelluloses. Suitable polyvinyl alcohols include those available from Celanese Corporation (Dallas, TX) under the Celvol trade name including, but not limited to, Celvol 523, Celvol 530, Celvol 540, Celvol 518, Celvol, 513, Celvol 508, Celvol 504, and combinations thereof. Suitable hydroxypropylmethylcelluloses include those available from the Dow Chemical Company (Midland, MI) under the Methocel trade name including, but not limited, to Methocel E50, Methocel E15, Methocel E6, Methocel E5, Methocel E3, Methocel F50, Methocel K100, Methocel K3, Methocel A400, and combinations thereof including combinations with above mentioned hydroxypropylmethylcelluloses.

In a particular embodiment, the above mentioned water-soluble polymer(s) of the present invention may be blended with any single starch or combination of starches as a filler material in such an amount as to reduce the overall level of water-soluble polymers required, so long as it helps provide the dissolvable porous solid with the requisite structure and physical/chemical characteristics as described herein. In such instances, the combined weight percentage of the water-soluble polymer(s) and starch-based material generally ranges from about 15% to about 40%, in one embodiment from about 15% to about 30%, and in a particular embodiment from about 15% to about 25% by weight relative to the total weight of the porous solid. The weight ratio of the water-soluble polymer(s) to the starch-based material can generally range from about 1:10 to about 10:1, in one embodiment from about 1:8 to about 8:1, in still another embodiment from about 1:7 to about 7:1, and in yet another embodiment from about 6:1 to about 1:6.

Typical sources for starch-based materials of the present invention can include cereals, tubers, roots, legumes and fruits. Native sources can include corn, pea, potato, banana, barley, wheat, rice, sago, amaranth, tapioca, arrowroot, canna, sorghum, and waxy or high amylase varieties thereof.

The starch-based materials of the present invention may also include native starches that are modified using any modification known in the art, including physically modified starches examples of which include sheared starches or thermally-inhibited starches; chemically modified starches including those which have been cross-linked, acetylated, and organically esterified, hydroxyethylated, and hydroxypropylated, phosphorylated, and inorganically esterified, cationic, anionic, nonionic, amphoteric and zwitterionic, and succinate and substituted succinate derivatives thereof; conversion products derived from any of the starches, including fluidity or thin-boiling starches prepared by oxidation, enzyme conversion, acid hydrolysis, heat or acid dextrinization, thermal and or sheared products may also be useful herein; and pregelatinized starches which are known in the art.

**Plasticizer**

The article may comprise a water soluble plasticizing agent suitable for use in compositions discussed herein. In one embodiment, the article comprises from about 1% to about 30% plasticizer, in another embodiment from about 3% to about 20%, and in yet another embodiment from about 5% to about 15%. Non-limiting examples of suitable plasticizing agents include polyols, copolyols, polycarboxylic acids, polyesters and dimethicone copolyols.

Examples of useful polyols include, but are not limited to, glycerin, diglycerin, propylene glycol, ethylene glycol, butylene glycol, pentylene glycol, cyclohexane dimethanol, hexane diol, polyethylene glycol (200-600), sugar alcohols such as sorbitol, manitol, lactitol and other mono-and polyhydric low molecular weight alcohols (e.g., $C_2$-$C_8$ alcohols); mono di- and oligosaccharides such as fructose, glucose, sucrose, maltose, lactose, and high fructose corn syrup solids and ascorbic acid.

Examples of polycarboxylic acids include, but are not limited to citric acid, maleic acid, succinic acid, polyacrylic acid, and polymaleic acid.

Examples of suitable polyesters include, but are not limited to, glycerol triacetate, acetylated-monoglyceride, diethyl phthalate, triethyl citrate, tributyl citrate, acetyl triethyl citrate, acetyl tributyl citrate.

Examples of suitable dimethicone copolyols include, but are not limited to, PEG-12 dimethicone, PEG/PPG-18/18 dimethicone, and PPG-12 dimethicone.

Other suitable platicizers include, but are not limited to, alkyl and allyl phthalates; napthalates; lactates (e.g., sodium, ammonium and potassium salts); sorbeth-30; urea; lactic acid; sodium pyrrolidone carboxylic acid (PCA); sodium hyraluronate or hyaluronic acid; soluble collagen; modified protein; monosodium L-glutamate; alpha & beta hydroxyl acids such as glycolic acid, lactic acid, citric acid, maleic acid and salicylic acid; glyceryl polymethacrylate; polymeric plasticizers such as polyquaterniums; proteins and amino acids such as glutamic acid, aspartic acid, and lysine; hydrogen starch hydrolysates; other low molecular weight esters (e.g., esters of $C_2$-$C_{10}$ alcohols and acids); and any other water soluble plasticizer known to one skilled in the art of the foods and plastics industries; and mixtures thereof.

Preferred placticizers include glycerin and propylene glycol. EP 0283165 B1 discloses other suitable plasticizers, including glycerol derivatives such as propoxylated glycerol. In some embodiments, the plasticizer is selected from the group consisting of glycerol, propylene glycol, butylene glycol, cyclohexane dimethanol and $C_2$-$C_8$ alcohols, alkyl and allyl phthallates, napthalates and esters of $C_2$-$C_{10}$ alcohols and acids, and mixtures thereof.

## Product Form

The dissolvable porous solids of the present invention can be produced in any of a variety of product forms, including dissolvable porous solids used alone or in combination with other personal care components. The dissolvable porous solids can be continuous or discontinuous when used in the personal care compositions. Regardless of the product form, the key to all of the product form embodiments contemplated within the scope of the method of the present invention is the selected and defined dissolvable porous solid that comprises a combination of a solid polymeric structurant and a surfactant-containing active ingredient.

The dissolvable porous solids of the present invention are preferably in the form of one or more flat sheets or pads of an adequate size to be able to be handled easily by the user. It may have a square, rectangle or disc shape or any other shape. The pads can also be in the form of a continuous strip including delivered on a tape-like roll dispenser with individual portions dispensed via perforations and or a cutting mechanism. Alternatively, the dissolvable porous solids of the present invention are in the form of one or more cylindrical objects, spherical objects, tubular objects or any other shaped object. The dissolvable porous solids of the present invention can have a thickness (caliper) of from about 0.5 mm to about 10 mm, in one embodiment from about 1 mm to about 9 mm, in another embodiment from about 2 mm to about 8 mm, and in still another embodiment from about 3 mm to about 7 mm. In the case of cylindrical, spherical, or other objects with more of a third dimension versus a pad or strip, the thickness is taken as the maximum distance of the shortest dimension, i.e., the diameter of a sphere or cylinder for instance.

The dissolvable porous solids of the present invention may comprise one or more textured, dimpled or otherwise topographically patterned surfaces including letters, logos or figures. The textured substrate preferably results from the shape of the substrate, in that the outermost surface of the substrate contains portions that are raised with respect to other areas of the surface. The raised portions can result from the formed shape of the article, for example the article can be formed originally in a dimpled or waffle pattern. The raised portions can also be the result of creping processes, imprinted coatings, embossing patterns, laminating to other layers having raised portions, or the result of the physical form of the dissolvable porous solid substrate itself. The texturing can also be the result of laminating the substrate to a second substrate that is textured.

In a particular embodiment, the dissolvable porous solids of the present invention can be perforated with holes or channels penetrating into or through the porous solid. These perforations can be formed during the drying process via spikes extended from the surface of the underlying mold, belt or other non-stick surface. Alternatively, these perforations can be formed after the drying process via poking or sticking the porous solids with pins, needles or other sharp objects. Preferably, these perforations are great in number per surface area, but not so great in number so as to sacrifice the integrity or physical appearance of the porous solid. It has been found that such perforations increase the dissolution rate of the porous solids into water relative to un-perforated porous solids.

The dissolvable porous solids of the present invention can also be delivered via a water insoluble implement or device. For instance, they may be attached or glued by some mechanism to an applicator to facilitate application to hair and/or skin, i.e., a comb, rag, wand, or any other conceivable water-insoluble applicator. Additionally, the dissolvable porous solids may be adsorbed to the surfaces a separate high surface area water-insoluble implement, i.e., a porous sponge, a puff, a flat sheet, etc. For the latter, the dissolvable porous solid of the present invention may be adsorbed as a film or layer.

**Product Types**

Non-limiting examples of product type embodiments for use by the dissolvable porous solids and methods of the present invention include hair conditioning substrates, moisturizing substrates, other hair treatment substrates, other skin or body treatment substrates, shaving preparation substrates, pet care substrates, personal care substrates containing pharmaceutical or other skin care active, moisturizing substrates, sunscreen substrates, chronic skin benefit agent substrates (e.g., vitamin-containing substrates, alpha-hydroxy acid-containing substrates, etc.), deodorizing substrates, fragrance-containing substrates, and the like.

I. Method of Manufacture

The personal care dissolvable porous solid articles of the present invention can be prepared by the process comprising: (1) Preparing a processing mixture comprising surfactant(s), dissolved polymer structurant, plasticizer and other optional ingredients; (2) Aerating the processing mixture by introducing a gas into the mixture to form an aerated wet mixture; (3) Forming the aerated wet mixture into a desired one or more shapes; (4) Drying the aerated wet mixture to a desired final moisture content (e.g., from about 0.1 to 25% moisture, by addition of energy); and (5) adding hydrophobic cosmetic active to the surface of the article.

**Preparation of Processing Mixture**

The processing mixture is generally prepared by dissolving the polymer structurant in the presence of water, plasticizer and other optional ingredients by heating followed by cooling. This can be accomplished by any suitable heated batch agitation system or via any suitable continuous system involving either single screw or twin screw extrusion or heat exchangers together with either high shear or static mixing. Any process can be envisioned such that the polymer is ultimately dissolved in the presence of water, the surfactant(s), the plasticizer, and other optional ingredients including step-wise processing via pre-mix portions of any combination of ingredients.

The processing mixtures of the present invention comprise from about 20% to about 50% solids, in another embodiment from about 25% to about 45% solids, and in another embodiment from about 30% to about 40% solids by weight of the processing mixture before drying; and have a viscosity of from about 5,000 centipoise ("cps") to about 150,000cps, in one embodiment from about 7,500 cps to about 125,000 cps, in another embodiment from about 10,000 cps to about 100,000 cps, and in still another embodiment from about 12,500 cps to about 75,000 cps .

The processing mixture viscosity values can be measured on a suitable rheometer, such as a TA Instruments AR500 Rheometer with 4.0 cm diameter parallel plate and 1,200 micron gap at a shear rate of 1.0 reciprocal seconds for a period of 30 seconds at 25°C (available from TA Instruments, New Castle, DE), or on a standard viscometer, such as a Brookfield Model DV-1 PRIME Digital Viscometer with CP-41 and CP-42 spindles at a shear rate of 1.0 reciprocal seconds for a period of 2 minutes at 25°C (available from Brookfield Engineering Laboratories, Inc., Middleboro, MA). The % solids content is the summation of the weight percentages by weight of the total processing mixture of all of the solid, semi-solid and liquid components excluding water and any obviously volatile materials such as low boiling alcohols.

**Aeration of Processing Mixture**

The aeration of the processing mixture is accomplished by introducing a gas into the mixture, preferably by mechanical mixing energy but also may be achieved via chemical means. The aeration may be accomplished by any suitable mechanical processing means, including but not limited to: (i) batch tank aeration via mechanical mixing including planetary mixers or other suitable mixing vessels, (ii) semi-continuous or continuous aerators utilized in the food industry (pressurized and non-pressurized), or (iii) spray-drying the processing mixture in order to form aerated beads or particles that can be compressed such as in a mould with heat in order to form the porous solid.

In a particular embodiment, it has been discovered that the dissolvable porous solids of the present invention can be prepared within semi-continuous and continuous pressurized aerators that are conventionally utilized within the foods industry in the production of marshmallows. Suitable pressurized aerators include the Morton whisk (Morton Machine Co., Motherwell, Scotland), the Oakes continuous automatic mixer (E.T. Oakes Corporation, Hauppauge, New York), the Fedco Continuous Mixer (The Peerless Group, Sidney, Ohio), and the Preswhip (Hosokawa Micron Group, Osaka, Japan).

The dissolvable porous solids of the present invention may also be prepared with chemical foaming agents by in-situ gas formation (e.g., via chemical reaction of one or more ingredients, including formation of $CO_2$ by an effervescent system).

**Forming the Aerated Wet Processing Mixture**

The forming of the aerated wet processing mixture may be accomplished by any suitable means to form the mixture in a desired shape or shapes including, but not limited to: (i) depositing the aerated mixture to specially designed moulds comprising a non-interacting and non-stick surface including Teflon, metal, HDPE, polycarbonate, neoprene, rubber, LDPE, glass and the like; (ii) depositing the aerated mixture into cavities imprinted in dry granular starch contained in a shallow tray (e.g., starch moulding forming technique widely utilized in the confectionery industry); and (iii) depositing the aerated mixture onto a continuous belt or screen comprising any non-interacting or non-stick material Teflon, metal, HDPE, polycarbonate, neoprene, rubber, LDPE, glass and the like which may be later stamped, cut, embossed or stored on a roll.

**Drying the Formed Aerated Wet Processing Mixture**

The drying of the formed aerated wet processing mixture may be accomplished by any suitable means including, but not limited to: (i) drying room(s) including rooms with controlled temperature and pressure or atmospheric conditions; (ii) ovens including non-convection or convection ovens with controlled temperature and optionally humidity; (iii) Truck/Tray driers, (iv) multi-stage inline driers; (v) impingement ovens; (vi) rotary ovens/driers; (vii) inline roasters; (viii) rapid high heat transfer ovens and driers; (ix) dual plenum roasters, (x) conveyor driers, (xi) microwave drying technology, and combinations thereof. Preferably, any suitable drying means that does not comprise freeze-drying can be used.

Optional ingredients may be imparted during any of the above described four processing steps or even after the drying process.

**Adding hydrophobic active to the surface of the article to form a Coated Article**

Any suitable application method can be used to apply the hydrophobic benefit agent to the article such that it forms a surface-resident coating that is adsorbed to at least a portion of the solid/air interface of the article. For instance, it can be sprayed, spread, dropped, printed, sandwiched between different articles or different portions of the same article, layered, injected, rolled on, or dipped. The hydrophobic benefit agent can be applied over portions or entire regions of the article's exterior surface, and can be applied in a manner to adorn, decorate, form a logo, design, etc.

II. Performance and Physical Characteristics

The article has a maximum Cell Wall Thickness. The article has a cell wall thickness of from about from about 0.02 mm to about 0.15 mm, in one embodiment from about 0.025 mm to about 0.12 mm, in another embodiment from about 0.03 mm to about 0.09 mm, and in still another embodiment from about 0.035 mm to about 0.06 mm.

The article has a minimum level of interconnectivity between the cells, which is quantified by both the Star Volume, the Structure Model Index (SMI), and the Percent Open Cell Content. The article has a Star Volume of from about 1 $mm^3$ to about 90 $mm^3$, in one embodiment from about 1.5 $mm^3$ to about 60 $mm^3$, in another embodiment from about 2 $mm^3$ to about 30 $mm^3$, and in still another embodiment from about 2.5 $mm^3$ to about 15 $mm^3$. The article has a non-negative Structure Model Index of from about 0.0 to about 3.0, in one embodiment from about 0.5 to about 2.75, and in another embodiment from about 1.0 to about 2.50. The article has a Percent Open Cell Content of from about 80% to 100%, in one embodiment from about 85% to about 97.5%, and in another embodiment from about 90% to about 95%.

The article also has a minimum Specific Surface Area. The article has a Specific Surface Area of from about 0.03 $m^2/g$ to about 0.25 $m^2/g$, in one embodiment from about 0.035 $m^2/g$ to about 0.22 $m^2/g$, in another embodiment from about 0.04 $m^2/g$ to about 0.19 $m^2/g$, and in still another embodiment from about 0.045 $m^2/g$ to about 0.16 $m^2/g$.

The article can have a basis weight of from about 125 grams/$m^2$ to about 3,000 grams/$m^2$, in one embodiment from about 200 grams/$m^2$ to about 2,500 grams/$m^2$, in another embodiment from about 300 grams/$m^2$ to about 2,000 grams/$m^2$, and in still another embodiment from about 400 grams/$m^2$ to about 1,500 grams/$m^2$.

The article has a solid density of from about 0.05 g/$cm^3$ to about 0.4 g/$cm^3$, in one embodiment from about 0.08 g/$cm^3$ to about 0.3 g/$cm^3$, in another embodiment from about 0.1 g/$cm^3$ to about 0.25 g/$cm^3$, and in another embodiment from about 0.12 g/$cm^3$ to about 0.2 g/$cm^3$.

**Dissolution Rate**

The dissolvable porous solid of present invention has a Dissolution Rate that allows the porous solid to rapidly disintegrate during use application with water. The Dissolution Rate of the dissolvable porous solid component is determined in accordance with the methodology described below.

Hand Dissolution Method: 0.5 to 0.8 g of the dissolvable porous solid (as described in the examples herein) is placed in the palm of the hand while wearing nitrile gloves. 7.5 $cm^3$ of luke warm tap water (from about 30°C to about 35°C) is quickly applied to the product via syringe. Using a circular motion, palms of hands are rubbed together 2 strokes at a time until dissolution occurs (up to 30 strokes). Undissolved material (after 30 strokes) is placed in preweighed weigh boat. Dry weight of undissolved material is measure the following day. The hand dissolution value is reported as the number of strokes it takes for complete dissolution or as 30 strokes as the maximum. For the latter scenario, the weight of the undissolved material is also reported.

The dissolvable porous solids of the present invention have a hand dissolution value of from about 1 to about 30 strokes, in one embodiment from about 2 to about 25 strokes, in another embodiment from about 3 to about 20 strokes, and in still another embodiment from about 4 to about 15 strokes.

## Thickness

The dissolvable porous solid of present invention is preferably a flat, flexible substrate in the form of a pad, a strip or tape and having a thickness of from about 0.5 mm to about 10 mm, in one embodiment from about 1 mm to about 9 mm, in another embodiment from about 2 mm to about 8 mm, and in a further embodiment from about 3 mm to about 7 mm as measured by the below methodology. In the case of cylindrical, spherical, or other objects with more of a third dimension versus a pad or strip, the thickness is taken as the maximum distance of the shortest dimension, i.e., the diameter of a sphere or cylinder for instance, and the thickness ranges are the same as described above.

The thickness of the dissolvable porous solid (i.e., substrate or sample substrate) is obtained using a micrometer or thickness gage, such as the Mitutoyo Corporation Digital Disk Stand Micrometer Model Number IDS-1012E (Mitutoyo Corporation, 965 Corporate Blvd, Aurora, IL, USA 60504). The micrometer has a 1 inch diameter platen weighing about 32 grams, which measures thickness at an application pressure of about 40.7 phi (6.32 $gm/cm^2$).

The thickness of the dissolvable porous solid is measured by raising the platen, placing a section of the sample substrate on the stand beneath the platen, carefully lowering the platen to contact the sample substrate, releasing the platen, and measuring the thickness of the sample substrate in millimeters on the digital readout. The sample substrate should be fully extended to all edges of the platen to make sure thickness is measured at the lowest possible surface pressure, except for the case of more rigid substrates which are not flat. For more rigid substrates which are not completely flat, a flat edge of the substrate is measured using only one portion of the platen impinging on the flat portion of the substrate.

## Basis Weight

The dissolvable porous solid component of the personal care composition of the present invention can have a basis weight of from about 125 $grams/m^2$ to about 3,000 $grams/m^2$, in one embodiment from about 150 $grams/m^2$ to about 1,200 $grams/m^2$, in another embodiment from about 200 $grams/m^2$ to about 2,500 $grams/m^2$, in a further embodiment from about 300 $grams/m^2$ to about 2,000 $grams/m^2$, and in still another embodiment from about 400 $grams/m^2$ to about 1,500 $grams/m^2$.

The Basis Weight of the dissolvable porous solid component of the personal care composition herein is calculated as the weight of the dissolvable porous solid component per area of the selected dissolvable porous solid ($grams/m^2$). The area is calculated as the projected area onto a flat surface perpendicular to the outer edges of the porous solid. For a flat object, the area is thus computed based on the area enclosed within the outer perimeter of the sample. For a spherical object, the area is thus computed based on the average diameter as 3.14 x $(diameter/2)^2$. For a cylindrical object, the area is thus computed based on the average diameter and average length as diameter x length. For an irregularly shaped three dimensional object, the area is computed based on the side with the largest outer dimensions projected onto a flat surface oriented perpendicularly to this side. This can be accomplished by carefully tracing the outer dimensions of the object onto a piece of graph paper with a pencil and then computing the area by approximate counting of the squares and multiplying by the known area of the squares or by taking a picture of the traced area (preferably shaded-in for contrast) including a scale and using image analysis techniques.

## Density

The dissolvable porous solid of the personal care compositions described herein can be characterized in terms of a density determination. In one embodiment the porous solid has a density of from about 0.05 $g/cm^3$ to about 0.4 $g/cm^3$, in a particular embodiment from about 0.08 $g/cm^3$ to about 0.3 $g/cm^3$, in another embodiment from about 0.10 $g/cm^3$ to about 0.25 $g/cm^3$, and in still another embodiment from about 0.12 $g/cm^3$ to about 0.20 $g/cm^3$.

The density of the dissolvable porous solid is determined by the equation: Calculated Density = Basis Weight of porous solid / (Porous Solid Thickness x 1,000).,

**Cell Inter-connectivity**

The dissolvable porous solid personal care products of the present invention with the above mentioned characteristics have a high degree of cell inter-connectivity, i.e., are predominantly open-celled solid foams as opposed to being predominantly closed-cell solid foams. The cell inter-connectivity can be assessed by light microscopy, scanning electron microscopy, micro computed tomography imaging parameters (Star Volume and SMI Index), gas pyncnometry parameters (% Open Cells), or other suitable methodology.

A preferred qualitative method of determining cell inter-connectivity is via light microscopy. This is performed by cutting a 2-3 mm wide sliver of the solid in the z-direction using scissors or a sharp blade, measured across the normal x-y largest surface of the solid, and turning the resulting sliver by 90 degrees to reveal the internal cellular structure of the freshly cut cross-sectional area. This cross-sectional area can be assessed by close visual inspection or, more accurately, by employing magnification under a stereo microscope such as the SZX12 Stereo microscope available from Olympus America Inc., Center Valley, PA. The open-celled dissolvable porous solids of the present invention can easily be identified by examining the inner portion of the cross-sectional area which will comprise a predominantly three dimensional network of struts with open void spaces surrounding the struts that are inter-connected to one another including in the third dimension through the depth of the cross-section. In contrast, the inner cross-section of a closed-cell foam will appear as discrete bubbles that are cut across and then only being inter-connected at the cross-sectional surface in two dimensions by virtue of the cutting process employed to generate the exposed cross-sectional area.

An expecially preferred and quantitative means to determine the cell interconnectivity is via the Star Volume and the Structure Model Index. Disk-like samples, approximately 4 cm in diameter and 3 to 7 mm high, are scanned using a micro computed tomography system (μCT80, SN 06071200, Scanco Medical AG). Each sample is imaged while sitting flat on the bottom of a cylindrical tube. Image acquisition parameters are 45 kVp, 177 μA, 51.2 mm field of view, 800 ms integration time, 1000 projections. The number of slices is adjusted to cover the height of the sample. The reconstructed data set consisted of a stack of images, each 2048x2048 pixels, with an isotropic resolution of 25 μm. For data analysis, a volume of interest is selected to be fully within the sample, avoiding the surface region. A typical volume of interest is 1028x772x98 voxels.

Structure Model Index (SMI) is measured using Scanco Medical's Bone Trabecular Morphometry evaluation with a threshold of 17. With this index the structural appearance of trabecular bone is quantified (*see* T. Hildebrand, P. Rüegsegger. Quantification of bone microarchitecture with the structure model index. Comp Meth Biomech Biomed Eng 1997;1:15-23). The triangulated surface is dilated in normal direction by an infinitesimal amount, and the new bone surface and volume is calculated. By this, the derivative of the bone surface (dBS/dr) can be determined. The SMI is then represented by the equation:

$$SMI = 6 \cdot \frac{BV \cdot \frac{dBS}{dr}}{BS^2}$$

SMI relates to the convexity of the structure to a model type. Ideal (flat) plates have an SMI of 0 (no surface change with dilation of the plates), whereas ideal cylindrical rods have an SMI of 3 (linear increase in surface with dilation of rods). Round spheres have an SMI of 4. Concave structure gives negative dBS/dr, resulting in negative SMI values. Artificial boundaries at the edge of the volume of interest are not included in the calculation and thus suppressed.

In addition to the Scanco Medical Analysis, StarVolume measurements are made. Star Volume is a measure of the "openness" of the void space in a two phase structure. By choosing a random uniformly distributed set of points in the phase of interest (in our case this is the background), we can extend lines in random directions from each of these points. The lines are extended until they touch the foreground phase. The length of each of these lines is then recorded. The random points have a sampling of 10 in each direction (x/y/z) and at each point 10 random angles are chosen. If the line extends to the border of the ROI of interest that line is discarded (we only want to accept lines that actually intersect with the foreground phase). The final equation is based upon the research published in Star volume in bone research. A histomorphometric analysis of trabecular bone structure using vertical sections; Vesterby, A.;Anat Rec.; 1993 Feb;235(2):325-334.:

$$StarVolume = \frac{4}{3}\pi \cdot \frac{\sum dist^3}{N}$$

where dist is the individual distances and N is the number of lines examined.

The Percent Open Cell Content is measured via gas pycnometry. Gas pycnometry is a common analytical technique that uses a gas displacement method to measure volume accurately. Inert gases, such as helium or nitrogen,

are used as the displacement medium. The sample is sealed in the instrument compartment of known volume, the appropriate inert gas is admitted, and then expanded into another precision internal volume. The pressure before and after expansion is measured and used to compute the sample volume. Dividing this volume into the sample weight gives the gas displacement density.

ASTM Standard Test Method D2856 provides a procedure for determining the percentage of open cells using an older model of an air comparison pycnometer. This device is no longer manufactured. However, you can determine the percentage of open cells conveniently and with precision by performing a test which uses Micromeritics' AccuPyc Pycnometer. The ASTM procedure D2856 describes 5 methods (A, B, C, D, and E) for determining the percent of open cells of foam materials.

For these experiments, the samples can be analyzed using an Accupyc 1340 using nitrogen gas with the ASTM foampyc software. Method C of the ASTM procedure is to be used to calculate to percent open cells. This method simply compares the geometric volume as determined using calipers and standard volume calculations to the true volume as measured by the Accupyc. It is recommended that these measurements be conducted by Micromeretics Analytical Services, Inc. (One Micromeritics Dr, Suite 200, Norcross, GA 30093). More information on this technique is available on the Micromeretics Analytical Services web sites (www.particletesting.com or www.micromeritics.com), or published in a book, "Analytical Methods in Fine particle Technology", by Clyde Orr and Paul Webb.

## Evaluation of Surface Resident Coating of Cosmetic Actives

The presence of a surface resident coating of a cosmetic active on the dissolvable porous solids of the present invention can be determined by a number of techniques. Cross-sections perpendicular to the larger surfaces of the foam can be examined by scanning electron microscopy (SEM) equipped with energy dispersive spectroscopy (EDS) capability. Other techniques for mapping silicon on the cross-sectional surface include: time of flight secondary ion mass spectroscopy (ToF-SIMS), and infrared spectroscopic.

A preferred method is to conduct elemental maps on cryo-SEM cross sections. Representative sections are cut from the porous solids with a clean razor blade and mounted with the freshly cut side facing upward on a standard cryo-SEM stub. The samples are secured onto the stub with carbon tape and silver paint. The samples are imaged using a Hitachi S-4700 FE-SEM fitted with a Gatan Alto 2500 cryo stage. The samples are cooled to -95°C before imaging in the microscope. The samples are lightly coated with Platinum to reduce charging. Representative images are collected at 2 kV, 20 uA extraction voltage, ultra high resolution mode using the lower secondary electron detector. Long working distances are used to allow large portions of the cross section (2-4 mm) to be imaged in one frame. Elemental mapping is performed using an Oxford Instruments energy-dispersive X-ray Spectrometer (Inca system). The mapping conditions are as follows: Accelerating voltage = 10 kV, Extraction voltage = 20uA, temperature = -105°C, Working distance = 12 mm (approximately), Condensor setting = 5. Elemental maps are collected for at least 200 seconds. The elements of interest (selecting an element present in the cosmetic active that is not present in the original porous solid before the cosmetic active is added) are then selected and displayed as a map.

The determination of a surface resident cosmetic active coating can be performed by comparing the distribution of the selected cosmetic active element across the cut cross-section of the porous solid. Specifically, the cosmetic active element should be present at the original solid/air interfaces (as a coating), but not within the exposed cross sectioned interior of the solid cell walls as can be ascertained by analyzing the exposed freshly cut cross-sectional interiors of the solid. It should be noted that some elemental contamination of the freshly cut cross-sectional solid cell wall interiors may occur as a consequence of the cutting process of the porous solid. However, the preponderance of the cosmetic active elemental distribution will still occur at the original solid/air interfaces and not within the exposed cut cross-sectional interiors of the cell walls as is exemplified herein.

It should also be noted that the surface resident cosmetic active coatings of the present invention generally do not spread uniformly across all exposed solid/air interfaces. Rather, it has been found that the surface resident coatings of the present invention typically spread, from the point of coating application, into cavities down to about 0.5 to 3 mm according to gravity. Accordingly, the determination of surface resident coatings of cosmetic actives of the present invention (as described above), should be conducted across many different cross sections from top-to-bottom and from edge-to-edge of the porous solid. The surface resident cosmetic active coatings will generally be within the regional vicinity (to within 0.5 to 3.0 mm from the surface) of the surface to where the cosmetic active was first applied.

## Cell Wall Thickness

The Cell Wall Thickness is computed from the scanned images via a micro computed tomography system ($\mu$CT80, SN 06071200, Scanco Medical AG) as described herein. The Cell Wall Thickness is determined according to the method defined for the measurement of Trabecular Thickness using Scanco Medical's Bone Trabecular Morphometry evaluation. The definition of Trabecular Thickness as taken from the Scanco User's manual: Trabecular Thickness uses

a Euclidean distance transformation (EDM), which calculates the Euclidean distance from any point in the foreground to the nearest background point. The Trabecular Thickness measure represents twice the centerline values associated with the local maxima of the EDM, which represents the distance to the center of the object (twice this distance will yield the thickness).

**Specific Surface Area**

The Specific Surface Area is measured via a gas adsorption technique. Surface Area is a measure of the exposed surface of a solid sample on the molecular scale. The BET (Brunauer, Emmet, and Teller) theory is the most popular model used to determine the surface area and is based upon gas adsorption isotherms. Gas Adsorption uses physical adsorption and capillary condensation to measure a gas adsorption isotherm. The technique is summarized by the following steps; a sample is placed in a sample tube and is heated under vacuum or flowing gas to remove contamination on the surface of the sample. The sample weight is obtained by subtracting the empty sample tube weight from the degassed sample + sample tube weight. The sample tube is then placed on the analysis port and the analysis is started. The first step in the analysis process is to evacuate the sample tube, followed by a measurement of the free space volume in the sample tube using helium gas at liquid nitrogen temperatures. The sample is then evacuated a second time to remove the helium gas. The instrument then begins collecting the adsorption isotherm by dosing krypton gas at user specified intervals until the requested pressure measurements are achieved.

Sample Preparation (Degassing): A sample not adequately cleaned of adsorbed contaminants will outgas during an analysis and some portion of the surface will be inaccessible to measurement. The purpose of degassing is to remove these adsorbed molecules from the surface of the sample prior to analysis. Adsorptive molecules must reach all parts of the surface for the true surface area to be revealed. Samples are prepared by heating the sample while simultaneously evacuating the sample tube.

For these experiments, the samples are outgassed under evacuation at room temperature overnight. Samples may then analyzed using an ASAP 2420 with krypton gas adsorption. Krypton gas is preferred over nitrogen gas as it has a saturation pressure approximately 1/300 that of nitrogen at liquid nitrogen temperature (krypton: 2.5 torr; nitrogen: 760 torr). Therefore, compared to nitrogen, there is in the free space above the sample about 1/300 the number of krypton molecules present at the same relative pressure. Since about the same number of krypton and nitrogen molecules are required to form a monolayer, this number represents a far greater proportion of the quantity dosed than in the case of nitrogen. It is recommended that these measurements be conducted by Micromeretics Analytical Services, Inc. (One Micromeritics Dr, Suite 200, Norcross, GA 30093). More information on this technique is available on the Micromeretics Analytical Services web sites (www.particletesting.com or www.micromeritics.com), or published in a book, "Analytical Methods in Fine particle Technology", by Clyde Orr and Paul Webb.

IV. Methods of Use

The compositions of the present invention may be used for treating mammalian keratinous tissue such as hair and/or skin, and provide rapid rinse-ability. The method for conditioning the hair may comprise the steps of: a) applying an effective amount of the dissolvable porous solid to the hand, b) wetting the dissolvable porous solid with water and rubbing to dissolve the solid, c) applying the dissolved material to either the hair or skin such as to treat, and d) rinsing the diluted treatment from the hair or skin using water. These steps can be repeated as many times as desired to achieve the desired treatment benefit.

V. Examples

The following examples further describe and demonstrate embodiments within the scope of the present invention. The examples are given solely for the purpose of illustration and are not to be construed as limitations of the present invention, as many variations thereof are possible without departing from the spirit and scope of the invention. All exemplified amounts are concentrations by weight of the total composition, i.e., wt/wt percentages, unless otherwise specified.

**Example 1: Concentrated surfactant premix preparation**

The following concentrated surfactant premix composition is prepared:

| Component | Wt% |
|---|---|
| Ammonium Laureth-3 sulfate (25% activity)[1] | 60.24 |

(continued)

| Component | Wt% |
|---|---|
| Ammonium Lauryl sulfate (25% activity)[2] | 36.14 |
| Cetyl alcohol[3] | 1.36 |
| Cocamide MEA[4] | 2.26 |
| Total | 100.0 |

[1] Ammonium Laureth-3 Sulfate at 25% active with an estimated C8/C9/C10/C11/C12/C13/C14/C15/C16/C18 alkyl chain length distribution of 0/0.3/0.9/0.5/71.5/1/20/1.9/3.9/0 and an average of 2.0 moles of ethoxylation, supplier: Procter & Gamble Chemicals
[2] Ammonium Lauryl Sulfate at 25% active with an estimated C8/C9/C10/C11/C12/C13/C14/C15/C16/C18 alkyl chain length distribution of 0/0.3/1.1/0.5/70.6/1.1/20.9/1.6/4.1/0, supplier: Procter & Gamble Chemicals
[3] CO-1694 CETYL ALCOHOL NF, supplier: Procter & Gamble Chemicals
[4] NINOL COMF, supplier: Stepan Company, Northfield, IL.

The ammonium laureth-3 sulfate and ammonium lauryl sulfate is added to a cleaned and tared vessel with stirring at 100-300 rpm with an overhead mixer. The mixture is slowly heated to 70-75C after which the cetyl alcohol and cocamide MEA are added. The mixture is allowed to again reach 70-75C while continuing to stir and then allowed to cool to room temperature. The actual product weight is then adjusted to the target product weight by adding additional distilled water (to compensate for evaporation during production) followed by stirring until homogenous.

**Example 2: Dissolving porous solid conditioning shampoo with a post-added silicone coating**

The following dissolving porous solid conditioning shampoo with a post-added silicone coating is prepared in accordance to the present invention:

| Component | Wt% |
|---|---|
| Surfactant premix from Example 1 | 67.0 |
| Polyvinyl alcohol[1] | 7.3 |
| Glycerin[2] | 2.9 |
| Perfume | 0.7 |
| Distilled water | 22.1 |
| Total | 100.0 |

[1] 87-89% hydrolyzed. 85,000 to 124,000 MW. Available from Sigma-Aldrich (St. Louis, MO 63178)
[2] Superol K, USP FCC EP Glycerin, supplier: Procter & Gamble Chemicals

A target weight of 250 grams of the above composition is prepared with the use of a commercial microwave oven (Sanyo EM-E1100S, Sanyo Electric., Ltd.) and a high speed mixing device (SpeedMixer™ DAC 400FV produced by Hauschild, Germany, and distributed by FlackTek Inc., Landrum, South Carolina). Distilled water and glycerin are added to a SpeedMixer™ Max 300 cup and mixed by rotating the closed container by hand until homogenous. The polyvinyl alcohol is added and the contents within the closed container are mixed at maximum speed (approximately 2,743 rounds per minute) for 30 seconds. Microwave heating is conducted in 15 to 40 second increments at a power level of 10 and with frequent stirrings via a spatula to prevent spill over. This procedure is repeated several times until the polyvinyl alcohol has been homogenously dispersed within the mixture. The closed container is then heated in a convection oven at 75 to 80C until the polyvinyl alcohol is fully dissolved (at least one hour). The surfactant premix (from Example 1) is then added to the polyvinyl alcohol/water/glycerin mixture and the resulting combination is mixed within the SpeedMixer™ at maximum speed for 30 seconds until homogenous. The perfume is then added followed by mixing for an additional 30 seconds with the SpeedMixer™ at maximum speed until homogenous. Deionized water is added until the target weight is achieved to compensate for evaporation during production) followed by mixing for an additional 30 seconds within the SpeedMixer™ at maximum speed until homogenous.

The above mixture is transferred into a 5 quart stainless steel bowl of a KitchenAid ® Mixer Model K5SS

(available from Hobart Corporation, Troy, OH) and fitted with a flat beater attachment. The mixture is vigorously aerated at high speed for approximately 90 seconds. The resulting aerated mixture is then spread with a spatula into 150 mm x 85 mm HDPE molds with a depth of 0.5 cm which are placed on a tray and then heated in a convection oven at a temperature of 75 to 80C for 30 minutes. The molds are then removed from the oven and placed into a second convection oven at a temperature of 40C for further drying overnight. The molds are removed from the oven and allowed to cool. The foam pads are then peeled away and removed from the molds and stored in sealed bags.

For performance testing, the foam samples are cut with scissors into 0.60 gram pieces on a four place balance and placed onto individual plastic weigh boats. Dimethicone with an average viscosity of 346,000 cps at 25C (CF330M from Momentive Performance Materials, Albany, New York) is applied to each piece at a target level of 0.027 grams on a suitable four place weight balance by brushing onto the surface with a small cosmetic brush applicator. If the target weight is exceeded, the excess is dimethicone is immediately removed via a small cosmetic sponge applicator. The samples are stored in bags overnight to enable the applied silicone to spread into the porous solid prior to testing. The dimethicone level is estimated to be approximately 4.3% by weight of the resulting porous solid (0.027 grams of dimethicone per 0.627 grams of porous solid).

**Comparative Example 3: Dissolving porous solid conditioning shampoo produced from a silicone emulsion**

The following dissolving porous solid conditioning shampoo produced from a silicone emulsion is not prepared in accordance to the present invention and is included only for comparative purposes:

| Component | Wt% |
|---|---|
| Surfactant premix from Example 1 | 67.0 |
| Polyvinyl alcohol[1] | 7.3 |
| Glycerin[2] | 2.9 |
| Perfume | 0.7 |
| Dimethicone[1] | 1.35 |
| Distilled water | 20.75 |
| Total | 100.0 |

[1] 87-89% hydrolyzed. 85,000 to 124,000 MW. Available from Sigma-Aldrich (St. Louis, MO 63178)
[2] Superol K, USP FCC EP Glycerin, supplier: Procter & Gamble Chemicals
[3] Dimethicone with an average viscosity of 346,000 cps at 25C available as CF330M from Momentive Performance Materials, Albany, New York.

A target weight of 250 grams of the above composition is prepared with the use of a commercial microwave oven (Sanyo EM-E1100S, Sanyo Electric., Ltd.) and a high speed mixing device (SpeedMixer™ DAC 400FV produced by Hauschild, Germany, and distributed by FlackTek Inc., Landrum, South Carolina) as described in Example 2, except with the addition of the dimethicone immediately after the addition of perfume to produce a dimethicone oil-in-water emulsion prior to the aeration and drying steps (versus being post-added to the resulting solid).

For performance testing, the foam samples are cut into 0.627 gram pieces. By assuming that all of the water is removed during the evaporation (to simplify the calculation), the dimethicone level is estimated to be approximately 4.38% by weight of the resulting porous solid (0.027 grams of dimethicone per 0.627 grams of porous solid).

**Example 4: Dissolving porous solid conditioning shampoo with cationic polymer and a post-added silicone coating**

The following dissolving porous solid conditioning shampoo with a post-added silicone coating is prepared in accordance to the present invention:

| Component | Wt% |
|---|---|
| Surfactant premix from Example 1 | 67.0 |
| Polyvinyl alcohol[1] | 7.3 |

(continued)

| Component | Wt% |
|---|---|
| Glycerin[2] | 2.9 |
| Perfume | 0.7 |
| Polyquaternium-10[3] | 0.5 |
| Distilled water | 21.6 |
| Total | 100.0 |

[1] 87-89% hydrolyzed. 85,000 to 124,000 MW. Available from Sigma-Aldrich (St. Louis, MO 63178)
[2] Superol K, USP FCC EP Glycerin, supplier: Procter & Gamble Chemicals
[3] UCARE™ Polymer LR400 (Code MB). Available from Amerchol Corporation (Plaquemine, Louisiana).

A target weight of 250 grams of the above composition is prepared with the use of a conventional overhead stirrer (IKA® RW20DZM Stirrer available from IKA® Works, Inc., Wilmington, DE), a commercial microwave oven (Sanyo EM-E1100S, Sanyo Electric., Ltd.) and a high speed mixing device (SpeedMixer™ DAC 400FV produced by Hauschild, Germany, and distributed by FlackTek Inc., Landrum, South Carolina). The distilled water is added to a glass beaker and is stirred via the overhead stirrer fitted with a 4 blade propeller at 100 to 200 rounds per minute. The polyquat-10 is then gradually added to the water while stirring and dissolved until the solution is clear forming a polyquat-10 premix. The polyquat-10 premix and glycerin is added to a tared SpeedMixer™ cup and mixed by rotating the closed container by hand until homogenous. The aeration and drying steps are completed as described in Example 2.

For performance testing, the foam samples are removed from the sealed bags and cut into 0.60 gram pieces. Dimethicone with an average viscosity of 346,000 cps at 25C (CF330M from Momentive Performance Materials, Albany, New York) is applied to each piece at a target level of 0.027 grams on a by brushing onto the surface with a small cosmetic brush applicator. If the target weight is exceeded, the excess is dimethicone is immediately removed via a small cosmetic sponge applicator. The samples are left on the weigh boats overnight to enable the applied silicone to absorb into the porous solid prior to testing. The dimethicone level is estimated to be approximately 4.3% by weight of the resulting porous solid (0.027 grams of dimethicone per 0.627 grams of porous solid).

**Example 5: Dissolving porous solid conditioning shampoo with cationic polymer produced from a silicone emulsion**

The following dissolving porous solid conditioning shampoo produced from a silicone emulsion is not prepared in accordance to the present invention and is included only for comparative purposes:

| Component | Wt% |
|---|---|
| Surfactant premix from Example 1 | 67.0 |
| Polyvinyl alcohol[1] | 7.3 |
| Glycerin[2] | 2.9 |
| Perfume | 0.7 |
| Polyquaternium-10[3] | 0.5 |
| Dimethicone[4] | 1.35 |
| Distilled water | 20.2 |
| Total | 100.0 |

[1] 87-89% hydrolyzed. 85,000 to 124,000 MW. Available from Sigma-Aldrich (St. Louis, MO 63178)
[2] Superol K, USP FCC EP Glycerin, supplier: Procter & Gamble Chemicals
[3] UCARE™ Polymer LR400 (Code MB). Available from Amerchol Corporation (Plaquemine, Louisiana).
[4] Dimethicone with an average viscosity of 346,000 cps at 25C available as CF330M from Momentive Performance Materials, Albany, New York.

A target weight of 250 grams of the above composition is prepared with the use of a conventional overhead

stirrer (IKA® RW20DZM Stirrer available from IKA® Works, Inc., Wilmington, DE), a commercial microwave oven (Sanyo EM-E1100S, Sanyo Electric., Ltd.) and a high speed mixing device (SpeedMixer™ DAC 400FV produced by Hauschild, Germany, and distributed by FlackTek Inc., Landrum, South Carolina). The distilled water is added to a glass beaker and is stirred via the overhead stirrer fitted with a 4 blade propeller at 100 to 200 rounds per minute. The polyquat-10 is then gradually added to the water while stirring and dissolved until the solution is clear forming a polyquat-10 premix. The polyquat-10 premix and glycerin is added to a tared SpeedMixer™ cup and mixed by rotating the closed container by hand until homogenous.

The remaining steps are performed as described in Example 2, except with the addition of the dimethicone immediately after the addition of perfume to produce a dimethicone oil-in-water emulsion prior to the aeration and drying steps (versus being post-added to the resulting solid).

For performance testing, the foam samples are removed from the sealed bags and cut into 0.627 gram pieces for testing. By assuming that all of the water is removed during the evaporation (to simplify the calculation), the dimethicone level is estimated to be approximately 4.38% by weight of the resulting porous solid (0.027 grams of dimethicone per 0.627 grams of porous solid).

**Example 6: Dissolving porous solid conditioning shampoo with cationic polymer and a post-added aminosilicone coating**

Dissolving porous solid shampoo pads are prepare according to the identical formulation and procedures as described in Example 4, but with the replacement of the dimethicone with an aminosilicone (Product code 65850 Y-14945 with a viscosity of 14,500cps at 25°C and an amine content of 0.050 meq/g).

**Examples 7-11: Conditioning performance and silicone deposition evaluation**

The above examples 2 through 5 are applied to hair with a shampooing protocol and evaluated for hair conditioning performance and the level of dimethicone deposited on hair (silicone deposition). The individual cut porous solid pieces from each example are applied to five 8 inch medium brown hair switches, each weighing 4 grams (i.e., 0.627 grams of solid applied to 20 grams of hair). The five switches are bound side-by-side together in a clamp and wetted with 100 degrees Fahrenheit tap water from a shower nozzle (1.5 grams of water per minute flow rate) while being hung in a stationary position over a sink. The cut porous solid piece is placed near the center of the hair switches and an additional 5 cubic centimeters of 100 degrees Fahrenheit tap water is applied on top of the solid piece via a plastic syringe. The wetted solid is rubbed slightly with the thumbs and then "milked" into the hair switches for 30 seconds with repeated up and down hand squeezes producing a wet lather. The lather is then thoroughly rinsed away from the hair switches by a 30 second rinsing with water flowing from the shower nozzle as described above. This application procedure is then repeated a second time. The five hair switches are then removed from the clamp and wrapped together with aluminum foil. The above treatment procedure is repeated for each of the four examples.

One switch from each group is removed from the foil and hung in a holder marked with the corresponding treatment identification. Panelists are asked to evaluate "ease of wet detangling" using the large end of a professional styling comb and then "ease of wet combing" using the small end of the same comb (on a 0 to 10 scale with 0 representing "hard" and 10 representing "easy"). The individual switches are left to hang and dry overnight and evaluated by the panelists for dry combing using only the small end of the comb. Evaluation of the "dry combing body" of the hair switch is performed separately from the evaluation of the "dry combing ends" of the hair switch (on a 0 to 10 scale with 0 representing "hard" and 10 representing "easy").

The resulting hair switches are then cut for deposition testing. Silicone is extracted from 1.5g of hair with 6 mL of 50:50 toluene:methylisobutyl ketone in 20 mL scintillation vials. The vials are agitated on a pulsed vortexer for 30 minutes. The silicone of the extract is quantified using inductively coupled plasma optical emission spectrometry (ICP-OES). ICP calibration standards of known silicone concentration are made using the same or a structurally comparable type of silicone raw material as the products being tested. The working range of the method is 8 - 2300 $\mu$g silicone per gram of hair. Data that has been collected representing examples 2 through 5 is given in the below table.

| Ex. | Porous Solid Description | Wet Detangling | Wet Combing | Dry Combing (Body) | Dry Combing (Ends) | Silicone deposition |
|---|---|---|---|---|---|---|
| 7 | Dissolving porous solid conditioning shampoo with a post-added silicone coating (Ex. 2) | 4.2 | 5.8 | 7.7 | 4.7 | 147 $\mu$g/g |

(continued)

| Ex. | Porous Solid Description | Wet Detangling | Wet Combing | Dry Combing (Body) | Dry Combing (Ends) | Silicone deposition |
|---|---|---|---|---|---|---|
| 8 | Dissolving porous solid conditioning shampoo produced from a silicone emulsion (Comparative Ex. 3) | 1.1 | 2.4 | 4.8 | 4.0 | 34 μg/g |
| 9 | Dissolving porous solid conditioning shampoo with cationic polymer and a post-added silicone coating (Ex. 4) | 6.4 | 7.7 | 7.4 | 5.7 | 107 μg/g |
| 10 | Dissolving porous solid conditioning shampoo with cationic polymer produced from a silicone emulsion (Comparative Ex. 5) | 3.5 | 5.5 | 4.3 | 4.4 | 56 μg/g |
| 11 | Dissolving porous solid conditioning shampoo with cationic polvmer and a post-added aminosilicone coating (Ex. 6) | 8.3 | 8.6 | 8.6 | 8.4 | 354 μg/g |

The above examples 7 through 11 demonstrate improved silicone deposition and correspondingly improved wet and dry conditioning performance for the porous solids produced according to the present invention (by post-adding the dimethicone onto the pre-formed porous solid as a coating via examples 2 and 4) relative to the porous solids produced not according the present invention (by emulsifying the dimethicone before the drying process to form the solid via examples 3 and 5). The former cited examples which were produced according to the present invention give increased estimated silicone deposition efficiencies of approximately 11% and 8%, respectively. The latter cited comparative examples that were produced contrary to the present invention give lower estimated silicone deposition efficiencies of approximately 2.5% and 4%, respectively. The last example also demonstrates excellent wet and dry conditioning performance and deposition efficiency from a post-added aminosilicone (produced in Example 6).

**Example 12: Evaluation of Surface Resident Silicone Coating**

The dissolving porous solid conditioning shampoo with a post-added silicone coating from Example 2 is analyzed via SEM imaging with elemental mapping of the element silicon (from the silicone) as described herein. Figures 1, 2, 3, and 4 show representative cross-sectional comparisons of the original SEM image on the left with the elemental map of silicone of the same cross-sectional view on the right (the presence of silicon shown with a white color). From these figures one can clearly see that the silicon (representative of silicone) resides predominantly at the porous solid/air interfaces versus being distributed throughout the interior of the freshly cut solid cell walls. These figures demonstrate the presence of the silicone cosmetic active as a surface resident coating across several solid/air interfaces (both at the upper surface and within exposed cavities within the solid). As described herein, it should be noted that the silicone coatings do not extend to all exposed solid/air interfaces. It can generally be seen that the silicone spreads into the cavities in close proximity to the surface of where the silicone was originally applied. Indeed, there are some cavities within these images where the silicone has not spread. It should be noted that these images 1 through 4 were chosen close to the surface where the silicone is applied so as to demonstrate the present invention. Images from the same sample taken from the opposite sided region generally do not show significant silicone accumulation at the interfaces. It can also be seen that there is some slight Si elemental contamination (or noise) across the images which is likely due to the cutting process. The significant differences in silicone accumulation can easily be seen above this level of contamination from the cutting process.

**Example 13: Representative Porous Solid Structural Measurements**

The following structural measurements were made of the porous solid produced in Example 2 (before the introduction of post-added silicone) according to the methods as described herein.

**Structural Characterization**

**(Porous Solid from Example 2 before introduction of post-added silicone)**

| Structural Parameter | Value |
|---|---|
| Kr BET Surface Area | 0.056 $m^2/g$ |
| Pycnometry % Open Cells | 96.5% |
| Micro-CT Cell Wall thickness | 0.112 mm |
| Micro-CT Star Volume | 43.7 $mm^3$ |
| Micro-CT SMI Index | 2.3 |

The above structural measurements are indicative of a predominantly open-celled porous solid and within the structural ranges of the present invention.

All documents cited are incorporated herein by reference in their entirety; the citation of any document is not to be construed as an admission that it is prior art with respect to the present invention. To the extent that any meaning or definition of a term in this document conflicts with any meaning or definition of the term in a document incorporated herein by reference, the meaning or definition assigned to the term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

Note that any actives and/or compositions disclosed herein can be used in and/or with the articles, and in particular the household care articles, disclosed in the following U.S Patent Applications, including any publications claiming priority thereto: US 61/229981; US 61/229986; US 61/229990; US 61/229996; US 61/230000; and US 61/230004. Such articles may comprise one or more of the following: detersive surfactant; plasticizer; enzyme; suds suppressor; suds booster; bleach; bleach stabilizer; chelant; cleaning solvent; hydrotrope; divalent ion; fabric softener additive (e.g. quaternary ammonium compounds); nonionic surfactant; perfume; and/or a perfume delivery system. Such articles may be utilized in methods including, but not limited to: dosing into a washing machine to clean and/or treat fabric; dosing into a dishwasher to clean and/or treat cutlery; and dosing into water to clean and/or treat fabric and/or hard surfaces.

## Claims

**1.** A flexible, non-lathering personal care article in the form of a porous dissolvable solid structure, comprising:

(a) from 0% to 10% ionic surfactant;
(b) from 1% to 60% of a hydrophobic surface-resident coating, wherein said coating comprises a non-surfactant cosmetic benefit agent;
(c) from 15% to 70% polymeric structurant, wherein said polymeric structurant has a weighted average molecular weight of from about 40,000 to about 500,000; and
(d) from 1% to 30% plasticizer;

wherein said article has a density of from about 0.05 $g/cm^3$ to about 0.4 $g/cm^3$; and wherein said coating has a Vaughan's solubility parameter VSP of from 0 $(cal/cm^3)^{1/2}$ to 10 $(cal/cm^3)^{1/2}$, alternatively from 1 $(cal/cm^3)^{1/2}$ to 10 $(cal/cm^3)^{1/2}$ , alternatively from 3 $(cal/cm^3)^{1/2}$ to 10 $(cal/cm^3)^{1/2}$, alternatively from 5 $(cal/cm^3)^{1/2}$ to 10 $(cal/cm^3)^{1/2}$.

**2.** A flexible, lathering personal care article in the form of a porous dissolvable solid structure, comprising:

(a) from 23% to 75% surfactant;
(b) from 1% to 60% of a hydrophobic surface-resident coating, wherein said coating comprises a non-surfactant cosmetic benefit agent;
(c) from 10% to 50% water soluble polymer; and

(d) from 1% to 30% plasticizer;

wherein said article has a density of from about 0.05 g/$cm^3$ to about 0.4 g/$cm^3$; and wherein said coating has a Vaughan's solubility parameter VSP of from 0 $(cal/cm^3)^{1/2}$ to 10 $(cal/cm^3)^{1/2}$, alternatively from 1 $(cal/cm^3)^{1/2}$ to 10 $(cal/cm^3)^{1/2}$, alternatively from 3 $(cal/cm^3)^{1/2}$ to 10 $(cal/cm^3)^{1/2}$, alternatively from 5 $(cal/cm^3)^{1/2}$ to 10 $(cal/cm^3)^{1/2}$.

**3.** A method for making the article of claim 1 or 2, comprising:

(a) Preparing a processing mixture comprising surfactant, dissolved polymer structurant, plasticizer and additional optional ingredients;
(b) Aerating the mixture by introducing a gas into the mixture to form an aerated wet mixture;
(c) Forming the aerated wet mixture into a desired one or more shapes;
(d) Drying the aerated wet mixture to a desired final moisture content to form a personal care article in the form of a porous dissolvable solid structure; and
(e) Applying a hydrophobic surface-resident coating that is adsorbed to the solid/air interface of said article, wherein said coating comprises a non-surfactant cosmetic active; and wherein said coating has a Vaughan's solubility parameter VSP of from 0 $(cal/cm^3)^{1/2}$ to 10 $(cal/cm^3)^{1/2}$, alternatively from 1 $(cal/cm^3)^{1/2}$ to 10 $(cal/cm^3)^{1/2}$, alternatively from 3 $(cal/cm^3)^{1/2}$ to 10 $(cal/cm^3)^{1/2}$, alternatively from 5 $(cal/cm^3)^{1/2}$ to 10 $(cal/cm^3)^{1/2}$.

**4.** The article of claim 1 or 2, wherein said hydrophobic coating has a Vaughan's solubility parameter of from 0 $(cal/cm^3)^{1/2}$ to 10 $(cal/cm^3)^{1/2}$, and wherein the article has a basis weight of from 125 grams/$m^2$ to 3,000 grams/$m^2$ and a thickness of from 0.5 mm to 10 mm.

**5.** The article of claim 1, 2, or 4, wherein said hydrophobic coating comprises silicone, petrolatum, hydrocarbon oil, natural or synthetic wax, paraffin, ozokerite, polyethylene, polybutene, polydecene, pentallydrosclualene, vegetable oil, triglyceride, perfume, or combinations thereof.

**6.** The article of claim 1, 2, or any of claims 4-5 that is substantially non-lathering and comprises from 1 % to 50% of a non-ionic surfactant, polymeric surfactant, or combination thereof.

**7.** The article of claim 1, 2, or any of claims 4-5, wherein the article comprises one or more water-soluble polymers such that their weighted average molecular weight is from about 40,000 to about 500,000.

## Patentansprüche

**1.** Elastischer, nicht schaumerzeugender Körperpflegegegenstand in Form einer porösen, auflösbaren, festen Struktur, umfassend:

(a) von 0 % bis 10 % ionisches Tensid;
(b) von 1 % bis 60 % eine hydrophobe, auf der Oberfläche verbleibende Beschichtung, wobei die Beschichtung einen nicht tensidischen, kosmetischen Wirkstoff umfasst;
(c) von 15 % bis 70 % polymeres Strukturmittel, wobei das polymere Strukturmittel ein durchschnittliches Molekulargewicht (Gewichtsmittel) von etwa 40.000 bis etwa 500.000 aufweist; und
(d) von 1 % bis 30 % Weichmacher;

wobei der Gegenstand eine Dichte von etwa 0,05 g/$cm^3$ bis etwa 0,4 g/$cm^3$ aufweist; und wobei die Beschichtung einen Vaughan-Löslichkeitsparameter VSP von 0 $(cal/cm^3)^{1/2}$ bis 10 $(cal/cm^3)^{1/2}$, als Alternative von 1 $(cal/cm^3)^{1/2}$ bis 10 $(cal/cm^3)^{1/2}$, als Alternative von 3 $(cal/cm^3)^{1/2}$ bis 10 $(cal/cm^3)^{1/2}$, als Alternative von 5 $(cal/cm^3)^{1/2}$ bis 10 $(cal/cm^3)^{1/2}$ aufweist.

**2.** Elastischer, schaumerzeugender Körperpflegegegenstand in Form einer porösen, auflösbaren, festen Struktur, umfassend:

(a) von 23 % bis 75 % Tensid;
(b) von 1 % bis 60 % eine hydrophobe, auf der Oberfläche verbleibende Beschichtung, wobei die Beschichtung einen nicht tensidischen, kosmetischen Wirkstoff umfasst;
(c) von 10 % bis 50 % wasserlösliches Polymer; und

(d) von 1 % bis 30 % Weichmacher;

wobei der Gegenstand eine Dichte von etwa 0,05 g/cm$^3$ bis etwa 0,4 g/cm$^3$ aufweist; und wobei die Beschichtung einen Vaughan-Löslichkeitsparameter VSP von 0 (cal/cm$^3$)$^{1/2}$ bis 10 (cal/cm$^3$)$^{1/2}$, als Alternative von 1 (cal/cm$^3$)$^{1/2}$ bis 10 (cal/cm$^3$)$^{1/2}$, als Alternative von 3 (cal/cm$^3$)$^{1/2}$ bis 10 (cal/cm$^3$)$^{1/2}$, als Alternative von 5 (cal/cm$^3$)$^{1/2}$ bis 10 (cal/cm$^3$)$^{1/2}$ aufweist.

**3.** Verfahren zur Herstellung des Gegenstands nach Anspruch 1 oder 2, umfassend:

(a) Herstellen einer Verarbeitungsmischung, umfassend Tensid, gelöstes Polymerstrukturmittel, Weichmacher und zusätzliche fakultative Bestandteile;
(b) Belüften der Mischung durch Einleiten eines Gases in die Mischung, um eine belüftete, nasse Mischung zu bilden;
(c) Ausbilden der belüfteten, nassen Mischung zu einer oder mehreren gewünschten Formen;
(d) Trocknen der belüfteten, nassen Mischung bis zu einem gewünschten endgültigen Feuchtigkeitsgehalt, um einen Körperpflegegegenstand in Form einer porösen, auflösbaren, festen Struktur zu bilden; und
(e) Auftragen einer hydrophoben, auf der Oberfläche verbleibenden Beschichtung, die an der Feststoff/Luft-Grenzfläche des Gegenstands adsorbiert wird, wobei die Beschichtung einen nicht tensidischen, kosmetischen Wirkstoff umfasst; und wobei die Beschichtung einen Vaughan-Löslichkeitsparameter VSP von 0 (cal/cm$^3$)$^{1/2}$ bis 10 (cal/cm$^3$)$^{1/2}$, als Alternative von 1 (cal/cm$^3$)$^{1/2}$ bis 10 (cal/cm$^3$)$^{1/2}$, als Alternative von 3 (cal/cm$^3$)$^{1/2}$ bis 10 (cal/cm$^3$)$^{1/2}$, als Alternative von 5 (cal/cm$^3$)$^{1/2}$ bis 10 (cal/cm$^3$)$^{1/2}$ aufweist.

**4.** Gegenstand nach Anspruch 1 oder 2, wobei die hydrophobe Beschichtung einen Vaughan-Löslichkeitsparameter von 0 (cal/cm$^3$)$^{1/2}$ bis 10 (cal/cm$^3$)$^{1/2}$ aufweist, und wobei der Gegenstand ein Basisgewicht von 125 Gramm/m$^2$ bis 3.000 Gramm/m$^2$ und eine Dicke von 0,5 mm bis 10 mm aufweist.

**5.** Gegenstand nach Anspruch 1, 2 oder 4, wobei die hydrophobe Beschichtung Silikon, Petrolatum, Kohlenwasserstofföl, natürliches oder synthetisches Wachs, Paraffin, Ozokerit, Polyethylen, Polybuten, Polydecen, Pentahydrosqualen, Pflanzenöl, Triglycerid, Duftstoff oder Kombinationen davon umfasst.

**6.** Gegenstand nach Anspruch 1, 2 oder einem der Ansprüche 4 bis 5, der im Wesentlichen nicht schaumerzeugend ist, und von 1 % bis 50 % ein nichtionisches Tensid, polymeres Tensid oder eine Kombination davon umfasst.

**7.** Gegenstand nach Anspruch 1, 2 oder einem der Ansprüche 4 bis 5, wobei der Gegenstand ein oder mehrere wasserlösliche Polymere derart umfasst, dass ihr durchschnittliches Molekulargewicht (Gewichtsmittel) von etwa 40.000 bis etwa 500.000 beträgt.

**Revendications**

**1.** Article de soin personnel souple non moussant sous la forme d'une structure solide soluble poreuse, comprenant :

(a) de 0 % à 10 % d'agent tensioactif ionique ;
(b) de 1 % à 60 % d'un revêtement hydrophobe situé en surface, dans lequel ledit revêtement comprend un agent à effet bénéfique cosmétique non tensioactif ;
(c) de 15 % à 70 % de structurant polymère, dans lequel ledit structurant polymère a une masse moléculaire moyenne pondérée allant d'environ 40 000 à environ 500 000 ; et
(d) de 1 % à 30 % de plastifiant ;

où ledit article a une masse volumique allant d'environ 0,05 g/cm$^3$ à environ 0,4 g/cm$^3$ ; et dans lequel ledit revêtement a un paramètre de solubilité de Vaughan (VSP) allant de 0 (cal/cm$^3$)$^{1/2}$ à 10 (cal/cm$^3$)$^{1/2}$, en variante de 1 (cal/cm$^3$)$^{1/2}$ à 10 (cal/cm$^3$)$^{1/2}$, en variante de 3 (cal/cm$^3$)$^{1/2}$ à 10 (cal/cm$^3$)$^{1/2}$, en variante de 5 (cal/cm$^3$)$^{1/2}$ à 10 (cal/cm$^3$)$^{1/2}$.

**2.** Article de soin personnel souple moussant sous la forme d'une structure solide soluble poreuse, comprenant :

(a) de 23 % à 75 % d'agent tensioactif ;
(b) de 1 % à 60 % d'un revêtement hydrophobe situé en surface, dans lequel ledit revêtement comprend un agent à effet bénéfique cosmétique non tensioactif ;

(c) de 10 % à 50 % de polymère hydrosoluble ; et
(d) de 1 % à 30 % de plastifiant ;

où ledit article a une masse volumique allant d'environ 0,05 g/$cm^3$ à environ 0,4 g/$cm^3$ ; et dans lequel ledit revêtement a un paramètre de solubilité de Vaughan (VSP) allant de 0 $(cal/cm^3)^{1/2}$ à 10 $(cal/cm^3)^{1/2}$, en variante de 1 $(cal/cm^3)^{1/2}$ à 10 $(cal/cm^3)^{1/2}$, en variante de 3 $(cal/cm^3)^{1/2}$ à 10 $(cal/cm^3)^{1/2}$, en variante de 5 $(cal/cm^3)^{1/2}$ à 10 $(cal/cm^3)^{1/2}$.

**3.** Procédé de fabrication de l'article selon la revendication 1 ou 2, comprenant :

(a) la préparation d'un mélange de traitement comprenant un agent tensioactif, un structurant polymère dissous, un plastifiant et des ingrédients facultatifs supplémentaires ;
(b) l'aération du mélange en introduisant un gaz dans le mélange pour former un mélange mouillé aéré ;
(c) la formation du mélange mouillé aéré en une ou plusieurs formes souhaitées ;
(d) le séchage du mélange mouillé aéré jusqu'à une teneur finale en humidité souhaitée pour former un article de soin personnel sous la forme d'une structure solide soluble poreuse ; et
(e) l'application d'un revêtement hydrophobe situé en surface qui est adsorbé sur l'interface solide/air dudit article, dans lequel ledit revêtement comprend un principe actif cosmétique non tensioactif ; et dans lequel ledit revêtement a un paramètre de solubilité de Vaughan (VSP) allant de 0 $(cal/cm^3)^{1/2}$ à 10 $(cal/cm^3)^{1/2}$, en variante de 1 $(cal/cm^3)^{1/2}$ à 10 $(cal/cm^3)^{1/2}$, en variante de 3 $(cal/cm^3)^{1/2}$ à 10 $(cal/cm^3)^{1/2}$, en variante de 5 $(cal/cm^3)^{1/2}$ à 10 $(cal/cm^3)^{1/2}$.

**4.** Article selon la revendication 1 ou 2, dans lequel ledit revêtement hydrophobe a un paramètre de solubilité de Vaughan allant de 0 $(cal/cm^3)^{1/2}$ à 10 $(cal/cm^3)^{1/2}$, et où l'article a une masse surfacique allant de 125 grammes/$m^2$ à 3000 grammes/$m^2$ et une épaisseur allant de 0,5 mm à 10 mm.

**5.** Article selon la revendication 1, 2 ou 4, dans lequel ledit revêtement hydrophobe comprend de la silicone, de la vaseline, une huile hydrocarbure, une cire naturelle ou synthétique, de la paraffine, de l'ozocérite, du polyéthylène, du polybutène, du polydécène, du pentahydrosqualène, une huile végétale, un triglycéride, un parfum, ou des combinaisons de ceux-ci.

**6.** Article selon la revendication 1, 2 ou l'une quelconque des revendications 4 à 5, qui est essentiellement non moussant et comprend de 1 % à 50 % d'un agent tensioactif non ionique, d'un agent tensioactif polymère, ou d'une combinaison de ceux-ci.

**7.** Article selon la revendication 1, 2 ou l'une quelconque des revendications 4 à 5, où l'article comprend un ou plusieurs polymères hydrosolubles de telle sorte que leur masse moléculaire moyenne pondérée va d'environ 40 000 à environ 500 000.

**Figure 1**

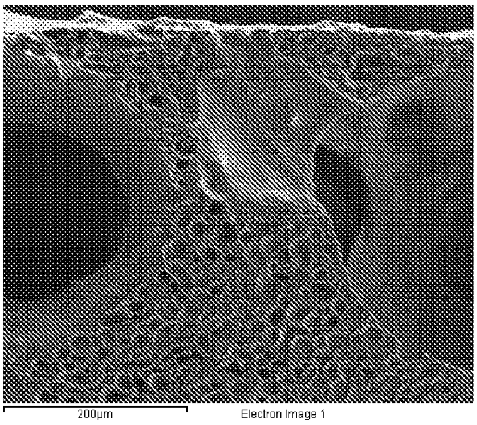


*SEM Image*

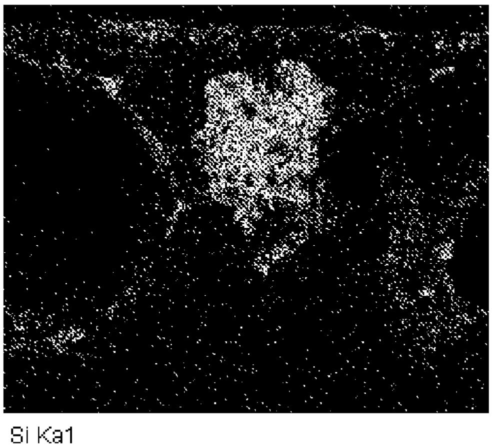


*Elemental Map of Silicon (white color)*

**Figure 2**

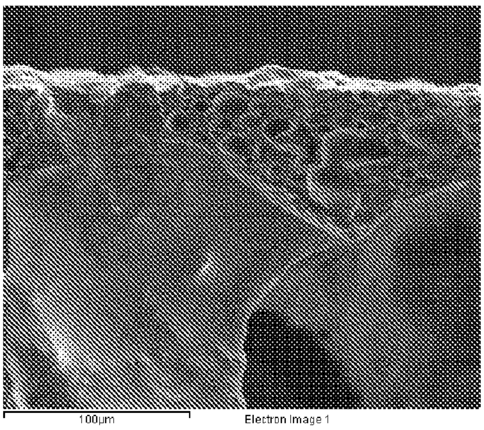


*SEM Image*

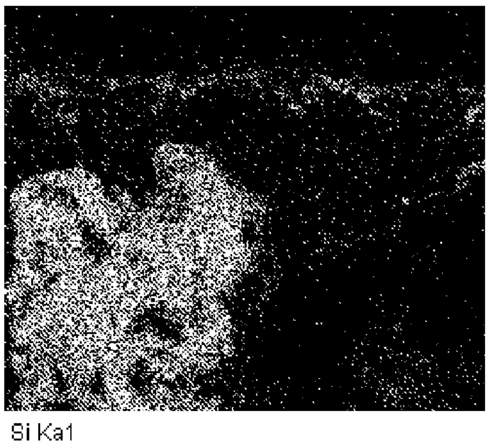


*Elemental Map of Silicon (white color)*

Figure 3

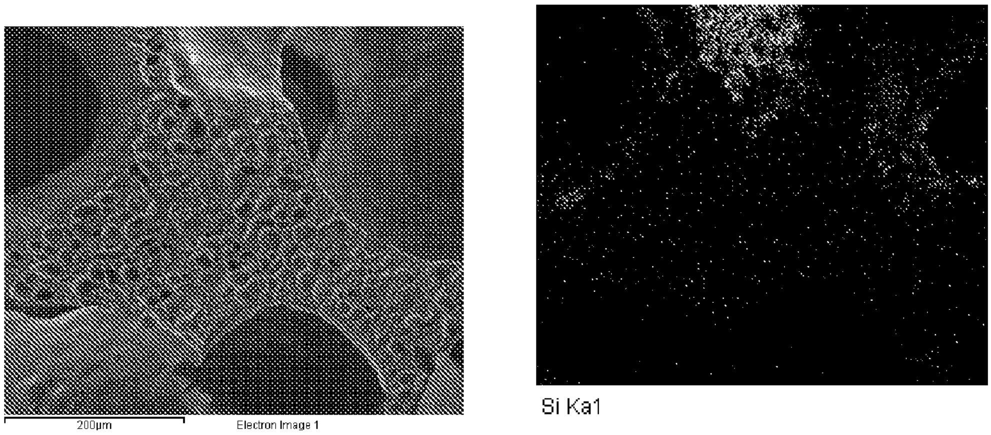


*SEM Image* *Elemental Map of Silicon (white color)*

Figure 4

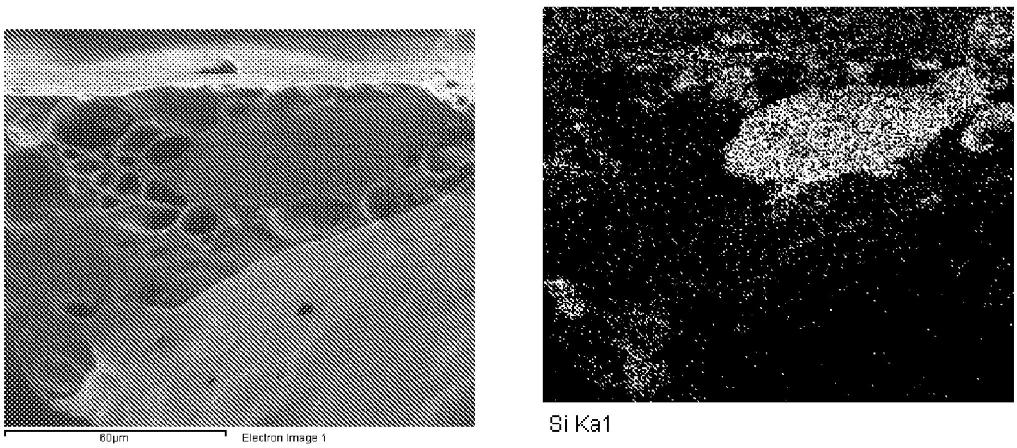


*SEM Image* *Elemental Map of Silicon (white color)*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 34584 A
- US 5104646 A
- US 5106609 A
- US 5674478 A
- US 5750122 A
- US 4529586 A, Clairol
- US 4507280 A, Clairol
- US 4663158 A, Clairol
- US 4197865 L, L'Oreal
- US 4217914 A, L'Oreal
- US 4381919 A, L'Oreal
- US 4422853 A, L'Oreal
- US 2809971 A
- US 3236733 A
- US 3753196 A
- US 3761418 A
- US 4345080 A
- US 4323683 A
- US 4379753 A
- US 4470982 A
- US 2694668 A
- US 3152046 A
- US 4089945 A
- US 4885107 A
- US 3929678 A, Laughlin
- US 2486921 A
- US 2486922 A
- US 2396278 A
- US 2658072 A
- US 2438091 A
- US 2528378 A
- US 4565647 A
- EP 0283165 B1
- US 60504 A
- US 61229981 B
- US 61229986 B
- US 61229990 B
- US 61229996 B
- US 61230000 B
- US 61230004 B


### Non-patent literature cited in the description


- **VAUGHAN.** *Cosmetics and Toiletries,* vol. 103
- **C. D. VAUGHAN ; COSMETICS ; TOILETRIES.** *Solubility, Effects in Product, Package, Penetration and Preservation,* October 1988, vol. 103
- Encyclopedia of Polymer Science and Engineering. John Wiley & Sons, Inc, 1989, vol. 15, 204-308
- McCutcheon's Detergents and Emulsifiers. Allured Publishing Corp, 1986
- McCutcheon's, Functional Materials. Allured Publishing Corp, 1992
- McCutcheon's, Emulsifiers and Detergents. M. C. Publishing Co, 1989
- McCutcheion's Detergents and Emulsifiers. Allured Publishing Corp, 1986
- McCutcheion's Functional Materials. 1992
- *Comp Meth Biomech Biomed Eng,* 1997, vol. 1, 15-23
- **VESTERBY, A.** Star volume in bone research. A histomorphometric analysis of trabecular bone structure using vertical sections. *Anat Rec.,* February 1993, vol. 235 (2), 325-334
- **CLYDE ORR ; PAUL WEBB.** Analytical Methods in Fine particle Technology